(19) Europäisches Patentamt
European Patent Office
Office européen des brevets

(11) EP 0 462 857 B2

(12) NOUVEAU FASCICULE DE BREVET EUROPEEN

(45) Date de publication et mention de la décision concernant l'opposition:
**04.03.1998 Bulletin 1998/10**

(45) Mention de la délivrance du brevet:
**17.08.1994 Bulletin 1994/33**

(21) Numéro de dépôt: **91401399.0**

(22) Date de dépôt: **30.05.1991**

(51) Int. Cl.$^6$: **A61K 7/13**, C07D 209/08, D06P 1/32, D06P 3/08, D06P 3/30

# (54) Composition tinctoriale à base de 5,6-dihydroxyindolines et procédé de teinture des fibres kératiniques

Färbemittel, enthaltend 5-6-Dihydroxyindoline und Färbungsverfahren für keratinische Fasern

Dyeing composition containing 5-6 dihydroxyindolines and keratinous fibres dyeing process

(84) Etats contractants désignés:
**AT BE CH DE DK ES FR GB GR IT LI NL SE**

(30) Priorité: **31.05.1990 FR 9006803**

(43) Date de publication de la demande:
**27.12.1991 Bulletin 1991/52**

(73) Titulaire: **L'OREAL**
**75008 Paris (FR)**

(72) Inventeurs:
- **Lagrange, Alain**
  **F-78400 Chatou (FR)**
- **Luppi, Bernadette**
  **F-93270 Sevran (FR)**
- **Junino, Alex**
  **F-93180 Livry-Gargan (FR)**

(74) Mandataire: **Casalonga, Axel et al**
**BUREAU D.A. CASALONGA - JOSSE**
**Morassistrasse 8**
**80469 München (DE)**

(56) Documents cités:

| | |
|---|---|
| WO-A-91/17739 | DE-A- 1 916 139 |
| GB-A- 726 078 | US-A- 4 013 404 |

- **S.T.N. SERVEUR DE BASES DE DONNEES, karlsruhe, DE, Fichier, Registry: RN=111233-48-8, & Fichier Chemical Abstracts, vol. 107, no. 23, résumé no. 214088m, Columbus, Ohio, US; J. ESCRIBANO et al.: "Kinetic study of the transient phase of a chemical reaction system coupled to an enzymically calatyzed step. Application to the oxidation of epinine by tyrosinase", & BIOPHYS. CHEM., 27(1), 15-25**
- **J. CHEM. SOC. part C, 1967, pages 1424-1427; S.N. MISHRA et al.: "Studies related to the chemistry of melanins. Part III. Synthesis of 5,6-dihydroxyindoline"**

EP 0 462 857 B2

## Description

La présente invention est relative à l'utilisation pour la teinture des fibres kératiniques, en particulier des fibres kératiniques humaines telles que les cheveux, de 5,6-dihydroxyindolines, aux compositions tinctoriales et aux procédés de teinture mettant en oeuvre ces composés.

On a déjà proposé dans le passé de teindre les cheveux en utilisant comme coupleurs, certaines monohydroxyindolines ou monoaminoindolines, notamment dans le brevet français n° 2 008 797; le brevet US-A-4 013 404 décrit des mono- ou diamino indolines ou des monohydroxyindolines comme base d'oxydation ou comme coupleurs, utilisés en teinture d'oxydation des cheveux.

On connaît par ailleurs les colorants de la famille des indoles, en particulier le 5,6-dihydroxy indole pour leur utilisation en teinture des fibres kératiniques telles que les cheveux humains, notamment par les brevets français FR-A-1 335 594 et 1 166 172.

On connait également, dans la demande de brevet internationale WO 91/17739, la 5,6-dihydroxyindoline et ses sels tels que le bromhydrate pour leur utilisation en teinture des fibres kératiniques telles que les cheveux, notamment en teinture par oxydation à l'air ou avec un système oxydant choisi parmi l'eau oxygénée, les périodates, le persulfate d'ammonium ou de potassium.

La demanderesse vient de découvrir que des 5,6-dihydroxyindolines, en particulier sous forme salifée, présentaient par rapport au 5,6-dihydroxy indole connu, des propriétés particulièrement remarquables en ce qui concerne la stabilité ou stockage dans les milieux habituellement utilisés en teinture des fibres kératiniques.

Ces composés présentent, en outre, une bonne solubilité dans l'eau par rapport au 5,6-dihydroxy indole.

Les inventeurs ont constaté par ailleurs que ces composés s'oxydaient particulièrement facilement en solution alcaline et pouvaient être utilisés en teinture capillaire sans éventuellement mettre en oeuvre un agent oxydant, ce qui permet d'obtenir une gamme variée de nuances plus ou moins intenses.

L'invention a donc pour objet l'utilisation pour la teinture des fibres kératiniques, en particulier des cheveux, de 5,6-dihydroxyindolines et de leurs sels.

Un autre objet de l'invention est constitué par les compositions tinctoriales destinées à la teinture des fibres kératiniques et en particulier des cheveux humains, contenant au moins une 5,6-dihydroxy indoline.

L'invention a également pour objet des procédés de teinture mettant en oeuvre ces composés et la 5,6-dihydroxyindoline. D'autres objets de l'invention apparaîtront à la lecture de la description et des exemples qui suivent.

Les 5,6-dihydroxyindolines utilisées pour la teinture des fibres kératiniques et en particulier des fibres kératiniques humaines telles que les cheveux, conformément à l'invention, sont essentiellement caractérisées par le fait qu'elles répondent à la formule :

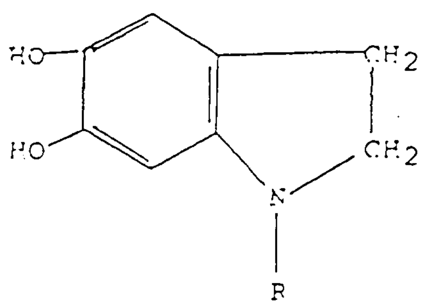

(I)

dans laquelle R représente un groupement alkyle $C_2$-$C_4$ et les sels d'addition d'acides de ces composés.

Les sels préférés sont les chlorhydrates ou bromhydrates.

Les composés préférés utilisés conformément à l'invention sont la N-éthyl 5,6-dihydroxyindoline, et la N-butyl 5,6-dihydroxyindoline.

La 5,6-dihydroxyindoline et les composés de formule (I) dans laquelle R est un groupement alkyle en $C_1$-$C_4$, sont synthétisés par déméthylation du 5,6-diméthoxyindole éventuellement N-alkylé, en présence d'HCl par chauffage à 140-150°C dans un tube scellé comme décrit dans J. Chem. Soc. (C) 1424 (1967) SWAN ou par chauffage au reflux dans HBr concentré à pression normale.

Les 5,6-diméthoxy N-alkyl indolines peuvent être préparées

- soit par alkylation classique de la 5,6-diméthoxyindoline à l'aide d'halogénure d'alkyle ou de sulfate de dialkyle,
- ou bien par réduction des dialcoxyindoles par le borohydrure de sodium en présence d'un acide carboxylique selon la méthode de GRIBBLL décrite dans J.A.C.S. - 1974, 96, 7812.

Les 5,6-dihydroxy N-alkyl indolines peuvent être préparées par alkylation directe de la 5,6-dihydroxyindoline qui peut être synthétisée selon la méthode de SWAN mentionnée ci-dessus.

Les N-alkyl($C_2$-$C_4$)5,6-dihydroxyindolines sont des composés nouveaux ainsi que leurs sels, et constituent un autre objet de l'invention.

Les 5,6-dihydroxyindolines de formule (I) définie ci-dessus, sont généralement mises en oeuvre à l'aide de compositions qui constituent un autre objet de l'invention.

Les compositions tinctoriales destinées à être utilisées pour la teinture des fibres kératiniques et en particulier les fibres kératiniques humaines telles que les cheveux, conformes à l'invention, sont caractérisées par le fait qu'elles contiennent dans un milieu approprié pour la teinture, au moins une 5,6-dihydroxy indoline répondant à la formule (I) définie ci-dessus.

L'invention a egalement pour objet une composition tinctoriale destinée à être utilisée pour la teinture des fibres kératinques en particulier des fibres kératiniques humaines contenant, dans un milieu approprié pour la teinture, comprenant de l'eau et au moins un solvant choisi parmi l'alcool éthylique, l'alcool propylique ou isopropylique, l'alcool tertiobutylique, l'éthylène glycol, les éthers mono méthyliques, monoéthyliques et monobutyliques de l'éthylène glycol, l'acétate du monoéthyl éther de l'éthylène glycol, le propylène glycol, les monométhyl éther du propylène glycol et du diproprylène glycol, le lactate de méthyle, au moins un composé de formule

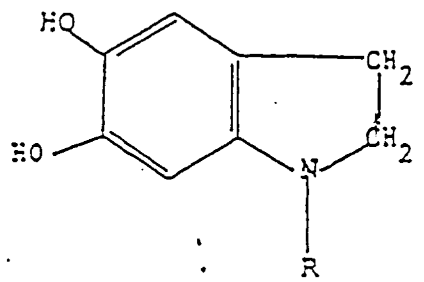

dans laquelle R désigne alkyle en $C_1$-$C_4$ ainsi que les sels d'addition d'acides de ces composés.

La quantité de 5,6-dihydroxyindoline(s) de formule (I) utilisées dans la composition, est comprise généralement dans des proportions de 0,01 à 8% en poids par rapport au poids total de la composition et de préférence de 0,03 à 5% en poids.

Ces compositions peuvent se présenter sous des formes diverses, notamment sous forme de lotions plus ou moins épaissies, de crèmes, de mousses et de gels, éventuellement conditionnées sous forme d'aérosols.

Elles peuvent également constituer un élément d'un agent de teinture à plusieurs composants disposé dans un dispositif à plusieurs compartiments ou kit de teinture.

Le milieu approprié pour la teinture est de préférence un milieu aqueux qui doit être cosmétiquement acceptable lorsque les compositions sont destinées à être utilisées pour la teinture des cheveux humains vivants. Ce milieu aqueux peut être constitué par de l'eau ou un mélange eau/solvant(s).

Le pH des compositions est compris entre 3 et 12.

Les solvants sont choisis parmi les solvants organiques et préférentiellement parmi l'alcool éthylique, l'alcool propylique ou isopropylique, l'alcool tertiobutylique, l'éthylèneglycol, les éthers monométhylique, monoéthylique et monobutylique de l'éthylèneglycol, l'acétate du monoéthyléther de l'éthylèneglycol, le propylèneglycol, les monométhyl éthers du propylèneglycol et du dipropylèneglycol, le lactate de méthyle.

Les solvants particulièrement préférés sont l'alcool éthylique et le propylèneglycol.

Les composés conformes à l'invention présentent l'avantage de pouvoir être utilisés dans un milieu essentiellement aqueux.

Il est également possible d'utiliser un milieu constitué de solvants anhydres choisis parmi les solvants définis ci-dessus. La composition est dans ce cas soit mélangée au moment de l'emploi avec un milieu aqueux, soit appliquée sur les fibres kératiniques mouillées au préalable par une composition aqueuse.

On appelle, conformément à l'invention, un milieu solvant anhydre, un milieu contenant moins de 1% d'eau.

Lorsque le milieu approprié pour la teinture est constitué par un mélange eau/solvant(s), les solvants sont utilisés dans des concentrations comprises entre 0,5 et 75% en poids par rapport au poids total de la composition, et de préférence dans des proportions inférieures à 20% en poids.

Les compositions conformes à l'invention peuvent contenir des adjuvants habituellement utilisés pour la teinture des fibres kératiniques et en particulier des adjuvants cosmétiquement acceptables lorsque ces compositions sont appliquées pour la teinture des cheveux humains vivants.

Ces compositions peuvent contenir notamment des amides gras dans des proportions préférentielles de 0,05 à

10% en poids, des agents tensio-actifs anioniques, cationiques, non ioniques, amphotères ou leurs mélanges présents de préférence dans des proportions comprises entre 0,1 et 50% en poids, des agents épaississants, des parfums, des agents séquestrants, des agents filmogènes, des agents de traitement, des agents dispersants, des agents de conditionnement, des agents conservateurs, des agents opacifiants, des agents de gonflement des fibres kératiniques.

Les agents épaississants sont choisis parmi l'alginate de sodium, la gomme arabique, la gomme de guar, les hétérobiopolysaccharides, tels que la gomme de xanthane, les scléroglucanes, les dérivés de cellulose, tels que la méthylcellulose, l'hydroxyéthylcellulose, l'hydroxypropylméthylcellulose, le sel de sodium de la carboxyméthylcellulose et les polymères d'acide acrylique réticulés de préférence.

On peut également utiliser les agents épaississants minéraux, tels que la bentonite.

Ces épaississants sont utilisés seuls ou en mélange et sont présents de préférence dans des proportions comprises entre 0,1 et 5% en poids par rapport au poids total de la composition et avantageusement entre 0,5 et 3% en poids.

Les agents alcalinisants utilisables dans les compositions peuvent être en particulier des amines, telles que les alcanolamines, des alkylamines, des hydroxydes ou carbonates alcalins ou d'ammonium.

Les agents d'acidification utilisables dans ces compositions peuvent être choisis parmi l'acide lactique, l'acide acétique, l'acide tartrique, l'acide phosphorique, l'acide chlorhydrique, l'acide citrique.

Il est bien entendu possible d'utiliser tout autre agent alcalinisant ou acidifiant acceptable, notamment dans le cas de la teinture des cheveux, en cosmétique.

Lorsque les compositions sont utilisées sous forme de mousse, elles peuvent être conditionnées sous pression et dans un dispositif aérosol en présence d'un agent propulseur et d'au moins un générateur de mousse.

Les agents générateurs de mousse peuvent être des polymères moussants anioniques, cationiques, non ioniques, amphotères ou leurs mélanges ou des agents tensio-actifs du type de ceux définis ci-dessus.

Le procédé de teinture des fibres kératiniques, en particulier des fibres kératiniques humaines, qui constitue un autre objet de l'invention, est essentiellement caractérisé par le fait qu'il consiste à appliquer sur ces fibres une composition (A) définie ci-dessus et contenant dans un milieu approprié pour la teinture, au moins une 5,6-dihydroxyindoline répondant à la formule (I')

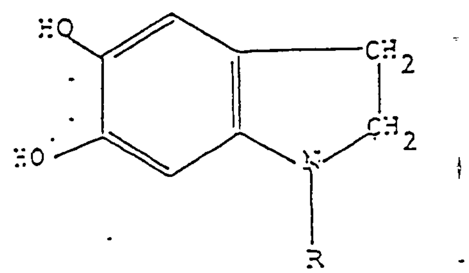

(I)

dans laquelle R représente hydrogène ou un groupement alkyle en $C_1$-$C_4$ ainsi que les sels d'addition d'acides de ces composés, à maintenir la composition au contact des fibres pendant un temps suffisant pour développer la coloration, soit à l'air, soit à l'aide d'un système oxydant, à rincer et éventuellement à laver les fibres ainsi teintes.

Selon une première forme de mise en oeuvre de l'invention, la coloration des fibres peut être effectuée sans addition d'un agent oxydant extérieur, au seul contact de l'air.

Selon une autre forme de réalisation, la couleur est révélée à l'aide d'un système oxydant chimique choisi parmi :

(i) des ions iodure et du peroxyde d'hydrogène, la composition (A) contenant la 5,6-dihydroxyindoline de formule (I') comprenant dans ce cas en plus, soit des ions iodure, soit du peroxyde d'hydrogène, et l'application de la composition (A) est précédée ou survie par l'application d'une composition (B) qui contient dans un milieu approprié pour la teinture, soit :

(a) du peroxyde d'hydrogène à un pH compris entre 2 et 12 et de préférence entre 2 et 7, lorsque la composition (A) contient des ions iodure, soit :
(b) des ions iodure a un pH compris entre 3 et 11 lorsque la composition (A) contient du peroxyde d'hydrogène;

(ii) l'application de la composition (A) contenant la 5,6-dihydroxyindoline de formule (I') étant suivie par l'application d'une composition aqueuse (B) présentant un pH acide, la composition (A) ou la composition (B) contenant au moins un nitrite;
(iii) des oxydants choisis parmi le peroxyde d'hydrogène, l'acide périodique et ses sels hydrosolubles, l'hypochlorite de sodium, la chloramine T, la chloramine B, le ferricyanure de potassium, l'oxyde d'argent, le réactif de Fenton,

l'oxyde de plomb (IV), le sulfate de cesium, le persulfate d'ammonium; ces oxydants étant présents dans la composition (A) contenant la 5,6-dihydroxyindoline de formule (I') ou appliqués simultanément ou séquentiellement au moyen d'une composition (B) les contenant dans un milieu approprié pour la teinture;

(iv) des anions d'un métal choisis parmi les permanganates ou bichromates, ces oxydants étant appliqués au moyen d'une composition (B) aqueuse, à un pH de 2 à 10, avant l'application de la composition (A);

(v) des sels métalliques des groupes 3 à 8 du tableau périodique, ces sels métalliques étant appliqués dans une étape séparée au moyen d'une composition (B) contenant ces sels dans un milieu approprié pour la teinture;

(vi) des sels de terres rares, ces sels de terres rares étant appliqués au moyen d'une composition (B) les contenant dans un milieu approprié pour la teinture, la composition (B) étant appliquée préalablement ou postérieurement à l'application de la composition (A) contenant la 5,6-dihydroxyindoline de formule (I');

(vii) un dérivé quinonique choisi parmi les ortho- ou parabenzoquinones, les ortho- ou parabenzoquinones monoimines ou diimines, les 1,2- ou 1,4-naphtoquinones, les ortho- ou parabenzoquinones sulfonimides, les $\alpha$, $\omega$-alkylène-bis-1,4-benzoquinones ou les 1,2-ou 1,4-naphtoquinones monoïmines ou diimines, la 5,6-dihydroxyindoline de formule (I') et les dérivés quinoniques étant choisis de façon à ce que la différence de potentiel d'oxydoréduction $\Delta E$ entre le potentiel d'oxydoréduction Ei de la 5,6-dihydroxyindoline de formule (I) déterminé à pH 7 en milieu phosphate sur électrode de carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leqq 320 \text{ millivolts};$$

la composition (B) étant appliquée préalablement ou postérieurement à l'application de la composition (A); sous réserve que lorsque le composé de formule (I) est la 5,6-dihydroxyindoline ou la n-méthyl 5,6-dihydroxyindoline ou l'un de ses sels d'addition d'acides d'acides le système oxydant est différent de l'air, du péroxyde d'hydrogène, de l'acide périodique et ses sels hydrosolubles, du persulfate d'ammonium.

Selon une forme préférée de l'invention, l'application des compositions (A) et (B) est séparée par une étape de rinçage à l'eau.

Selon une première variante du procédé de teinture mettant en oeuvre des systèmes oxydants, on applique sur les matières kératiniques une composition (A) contenant dans un milieu approprié pour la teinture, au moins un composé de formule (I') en association avec des ions iodure, l'application de la composition (A) étant précédée ou suivie par l'application d'une composition (B) qui contient dans un milieu approprié pour la teinture, du peroxyde d'hydrogène.

Ce procédé peut également être mis en oeuvre en appliquant sur les fibres kératiniques au moins une composition (A) contenant dans un milieu approprié pour la teinture, le composé de formule (I') en association avec du peroxyde d'hydrogène, ayant un pH compris entre 2 et 7 et de préférence entre 3,5 et 7, l'application de la composition (A) étant précédée ou suivie par l'application d'une composition (B) qui contient dans un milieu approprié pour la teinture, des ions iodure.

L'ion iodure dans cette variante du procédé, est choisi de préférence parmi les iodures de métaux alcalins, alcalino-terreux ou d'ammonium. L'iodure est plus particulièrement constitué par l'iodure de potassium.

Les ions iodure sont présents dans les compositions (A) ou (B) dans des proportions comprises généralement entre 0,007 et 4% en poids exprimées en ions $I^-$ et de préférence entre 0,08 et 1,5% en poids par rapport au poids total de la composition (A) ou (B).

Selon une seconde variante, ce procédé peut être mis en oeuvre en utilisant comme agent oxydant pour révéler la coloration, un nitrite. Les nitrites plus particulièrement utilisables conformément à l'invention, sont :

- des nitrites de métaux alcalins, alcalino-terreux ou d'ammonium ou de tout autre cation cosmétiquement acceptable lorsqu'il est utilisé pour la teinture des cheveux humains vivants;
- des dérivés organiques des nitrites tels que par exemple le nitrite d'amyle;
- des vecteurs de nitrites, c'est-à-dire des composés qui par transformation forment des nitrites du type défini ci-dessus.

Les nitrites particulièrement préférés sont les nitrites de sodium, de potassium ou d'ammonium.

Cette variante du procédé est mise en oeuvre en appliquant sur les matières kératiniques, la composition (A) à base du composé de formule (I') définie ci-dessus, puis une composition aqueuse acide (B), la composition (A) ou (B) contenant au moins un nitrite.

Les nitrites sont généralement utilisés dans des proportions comprises entre 0,02 et 1 mole/litre.

Selon une troisième variante de ce procédé, les oxydants sont choisis parmi le peroxyde d'hydrogène, la chloramine T, la chloramine B, l'acide periodique et ses sels hydrosolubles, l'hypochlorite de sodium, le ferricyanure de potassium, l'oxyde d'argent, le réactif de Fenton, l'oxyde de plomb (IV), le sulfate de césium, le persulfate d'ammonium. Ces agents sont de préférence appliqués sur les fibres au moyen d'une composition (B) et après l'application de la composition (A).

Ces agents oxydants sont présents dans des proportions suffisantes pour développer une coloration et de préférence dans des proportions comprises entre 0,004 mole et 0,7 mole, en particulier entre 0,01 mole et 0,04 mole pour 100 g de composition.

Selon une quatrième variante de ce procédé, on applique dans un premier temps sur les fibres kératiniques, une composition contenant dans un milieu approprié pour la teinture, à un pH compris entre 2 et 10, un anion d'un métal ayant une bonne affinité pour la kératine ayant un potentiel d'oxydoréduction supérieur à celui des composés de formule (I'). Cet anion est choisi de préférence parmi les permanganates ou les bichromates et plus particulièrement le permanganate de potassium et le bichromate de sodium.

Ces anions métalliques sont généralement utilisés à des molalités en anions supérieures à $10^{-3}$ moles/1000 g jusqu'à de préférence de 1 mole/1000 g.

Dans un second temps, on applique une composition contenant dans un milieu approprié pour la teinture, à un pH compris entre 4 et 10, un composé répondant à la formule (I') définie ci-dessus.

Les compositions contenant les anions ne doivent pas contenir d'agents organiques ayant un effet réducteur sur les anions.

Selon une cinquième variante de l'invention, on met en oeuvre des catalyseurs d'oxydation choisis parmi les sels métalliques tels que les sels de manganèse, de cobalt, de fer, de cuivre et d'argent.

A titre d'exemple, on peut utiliser le sulfate de manganèse, le lactate de manganèse, le chlorure de cobalt, le chlorure ferrique, le chlorure cuivrique, le nitrate d'argent ammoniacal.

Les sels préférés sont les sels de cuivre. Ces sels sont utilisés dans des proportions de 0,01 à 2% exprimées en ions métalliques par rapport au poids total de la composition mise en oeuvre et contenant ces sels.

Selon cette variante, on met les fibres kératiniques, en particulier les cheveux, en contact avec une composition (B) contenant dans un milieu approprié pour la teinture, le sel métallique, avant ou après l'application de la composition (A) contenant le composé de formule (I') et on rince de préférence entre les deux étapes.

La forme de réalisation préférée consiste à appliquer un sel cuivrique dans un premier temps et la composition (A) contenant la 5,6-dihydroxyindoline de formule (I'), dans un deuxième temps.

On peut faire suivre cette teinture après rinçage, de l'application d'une solution de peroxyde d'hydrogène pour éventuellement éclaircir la couleur obtenue.

Selon une sixième variante, on utilise des sels de terres rares. Les sels de terres rares utilisables conformément à l'invention sont choisis parmi les sels de Lanthanides, et notamment les sels de Cérium $Ce^{3+}$, $Ce^{4+}$, de Lanthane $La^{3+}$, d'Europium $Eu^{2+}$, $Eu^{3+}$, de Gadolinium $Gd^{3+}$, d'Ytterbium $Yb^{2+}$, $Yb^{3+}$, de Dysprosium $Dy^{3+}$. Les sels préférés sont en particulier les sulfates, chlorures ou nitrates.

Ces sels de terres rares sont présents dans des proportions comprises entre 0,1 et 8% en poids par rapport au poids total de la composition.

On utilise de préférence les sels de Cérium $Ce^{3+}$ et $Ce^{4+}$ sous la forme de sulfates et de chlorures.

Selon une septième variante, la composition contenant le dérivé quinonique est appliquée avant ou après la composition (A) contenant le composé de formule (I').

A titre d'exemple de dérivé quinonique, on peut citer la 1,4-benzoquinone.

La concentration en dérivés quinoniques est de préférence comprise entre 0,005 et 1 mole/litre dans la composition (B). Le pH de la composition (B) est compris entre 2 et 10 et de préférence inférieur à 7.

Lorsqu'on met en oeuvre des compositions à base de peroxyde d'hydrogène dans les différents procédés décrits ci-dessus, la teneur en peroxyde d'hydrogène est généralement comprise entre 1 et 40 volumes et de préférence entre 2 et 10 volumes et plus particulièrement entre 3 et 10 volumes.

Dans une forme de realisation préferée le milieu approprié pour la teinture comprend de l'eau et au moins un solvant choisi parmi l'alcool éthylique, l'alcool propylique ou isopropylique, l'alcool tetriobutylique, l'éthylène glycol, les éthers mono méthyliques, monoéthyliques et monobutyliques de l'éthylène glycol, l'acétate du monoéthyl éther de l'éthylène glycol, le propylène glycol, les monométhyl éther du propylène glycol et du dipropylène glycol, le lactate de méthyle,

L'invention a également pour objet un agent de coloration des fibres kératiniques et en particulier des fibres kératiniques humaines, à plusieurs composants, destiné notamment à être utilisé dans la mise en oeuvre du procédé de teinture défini ci-dessus et utilisant un système oxydant. Dans ce cas, l'agent de teinture comprend au moins deux composants dont un premier est constitué par la composition (A) définie ci-dessus et contenant la 5,6-dihydroxyindoline de formule (I') et l'autre composant est constitué par l'une des compositions (B) également définie ci-dessus.

Les composants (A) et (B) respectifs sont choisis selon les différentes variantes du procédé exposées ci-dessus.

L'invention a également pour objet un dispositif à plusieurs compartiments ou encore "kit de teinture" ou "nécessaire de teinture", comportant tous les composants destinés à être appliqués dans une même teinture sur les fibres

kératiniques en applications uniques ou successives avec ou sans prémélange comme mentionné ci-dessus.

De tels dispositifs sont connus en eux-mêmes et peuvent comporter un premier compartiment contenant la composition (A) contenant la 5,6-dihydroxyindoline de formule (I') dans un milieu approprié pour la teinture, et dans un second compartiment une composition (B) du type défini ci-dessus et contenant l'agent oxydant tel que defini ci-dessus. Les dispositifs à plusieurs compartiments utilisables conformément à l'invention, peuvent être équipés des moyens de mélanges au moment de l'emploi et leur contenu peut être conditionné sous atmosphère inerte.

Lorsque le milieu contenant la 5,6-dihydroxy indoline de formule (I') est anhydre, on peut prévoir un troisième compartiment contenant un milieu aqueux approprié pour la teinture et destiné à être mélangé tout juste avant l'emploi avec la composition du premier compartiment.

La 5,6-dihydroxyindoline de formule (I'), les compositions et le procédé conformes à l'invention, peuvent être mis en oeuvre pour teindre des cheveux naturels ou déjà teints, permanentés ou non, défrisés ou non, ou des cheveux fortement ou légèrement décolorés et éventuellement permanentés.

Il est également possible de les utiliser pour la teinture de la fourrure ou de la laine.

Les exemples sont destinés à illustrer l'invention.

EXEMPLE DE PREPARATION 1

Préparation du bromhydrate de la N-butyl 5,6-dihydroxy indoline.

1ère étape:

Préparation de la N-butyl 5,6-diméthoxyindoline.

30 g (0,17 mole) de 5,6-diméthoxyindoline sont mis en solution dans 300 ml de diméthoxyéthane, en présence de 10,8 g (0,17 mole) de KOH et 500 mg de tétrabutylammonium hydrogénosulfate.

On ajoute rapidement 18 ml (0,17 mole) de bromobutane et on chauffe au reflux pendant 5 heures. Le mélange réactionnel est ensuite versé dans de l'eau. Après extraction au chlorure de méthylène et évaporation du solvant, on obtient un liquide coloré qui est purifié par chromatographie sur gel de silice, en utilisant comme éluant du chlorure de méthylène.

On obtient 27,6 g de N-butyl 5,6-diméthoxy indoline se présentant sous forme de liquide incolore.

2ème étape :

Préparation du bromhydrate de N-butyl 5,6-dihydroxy indoline.

5 g (0,02 mole) de N-butyl 5,6-diméthoxy indoline dans 50 ml d'acide bromhydrique à 48%, sont chauffés au reflux pendant 3 heures.

La solution est évaporée à sec et le résidu, dissous dans 100 ml d'éthanol absolu en présence de noir végétal, est chauffé au reflux pendant 30 minutes.

Après filtration sur célite et concentration sous vide, le produit est recristallisé dans l'éthanol absolu.

On obtient 3,5 g de bromhydrate de N-butyl 5,6-dihydroxyindoline se présentant sous forme de cristaux brun clair.

Dosage du bromure = 3,31 meq/g
Théorie : 3,47 meq/g.

EXEMPLE DE PREPARATION 2

Préparation de la N-éthyl 5,6-dihydroxyindoline (bromhydrate).

4,5 g (0,02 mole) de N-éthyl 5,6-diméthoxy indoline dans 40 ml d'acide bromhydrique à 48%, sont chauffés au reflux pendant 3 heures.

La solution est évaporée à sec et le résidu, dissous dans 80 ml d'éthanol absolu en présence de noir végétal, est chauffé au reflux pendant 30 minutes.

Après filtration sur célite et concentration sous vide, le produit est précipité par addition d'éther éthylique.

On obtient 4,7 g de bromhydrate de N-éthyl 5,6-dihydroxyindoline se présentant sous forme de solide jaune orangé.

Dosage du bromure = 3,87 meq/g

Théorie : 3,84 meq/g.

EXEMPLE 1

COMPOSITION (B5)

| | |
|---|---|
| - Sulfate de cuivre pentahydraté | 1,0 g |
| - Laurylsulfate de sodium vendu sous la dénomination SIPON LCS98 par la Société HENKEL | 1,0 g |
| - Monoéthanolamine qs | pH 9 |
| - Eau qsp | 100,0 g |

On applique cette composition pendant 5 minutes sur des cheveux gris permanentés. Après rinçage, on applique la composition (A5) suivante :

COMPOSITION (A5)

| | |
|---|---|
| - Bromhydrate de 5,6-dihydroxyindoline | 0,78 g |
| - Alcool éthylique à 96° | 10,0 g |
| - Hydroxypropylcellulose vendue sous la dénomination KLUCEL G par la Société AQUALON | 2,0 g |
| - Alkyléther de glycoside vendu à 60% de MA sous la dénomination TRITON CG 110 par ROHM & HAAS | 2,1 g MA |
| - Monylphénol oxyéthyléné à 9 moles d'oxyde d'éthylène | 0,1 g |
| - Acide tartrique | 0,3 g |
| - Triéthanolamine qs | pH 8,5 |
| - Eau qsp | 100,0 g |

Après avoir laissé poser cette composition pendant 10 minutes, les cheveux sont rincés puis séchés. Ils sont colorés en brun cendré.

EXEMPLE 2

COMPOSITION (A7)

| | |
|---|---|
| - Bromhydrate de 5,6-dihydroxyindoline | 1,6 g |
| - Alcool éthylique à 96° | 10,0 g |
| - Iodure de potassium | 0,8 g |
| - Hydroxyéthylcellulose vendue sous la dénomination NATROSOL 250 HHR par la Société AQUALON | 1,0 g |
| - Alkyléther de glycoside vendu sous la dénomination TRITON CG 110 par la Société ROHM & HAAS | 5,0 g MA |
| - Lauryléther sulfate de sodium | 0,2 g MA |
| - Triéthanolamine qs | pH 6,5 |
| - Eau qsp | 100,0 g |

Cette composition est appliquée sur des cheveux gris à 90% de blancs pendant 15 minutes. Après rinçage, on applique une solution d'eau oxygénée à 12,5 volumes pendant 5 minutes. Après rinçage, lavage et séchage, les cheveux sont colorés en blond foncé cendré.

EXEMPLE 3

On prépare une solution à 2,5% de bromhydrate de 5,6-dihydroxyindoline en milieu hydroalcoolique 90/10. On laisse cette solution au contact d'une mèche de cheveux à 90% de blancs pendant 15 minutes à température ambiante. On rince, on essore, puis on révèle la coloration par une solution hydroalcoolique 60/40 à 2% de 1,4-benzoquinone.

Le temps de pose est de 8 minutes.

Après rinçage, shampooing, rinçage et séchage, on obtient une coloration noire.

Les valeurs des potentiels d'oxydo-réduction sont :

$$E_I = 100 \text{ mV} \qquad Eq = 10 \text{ mV} \qquad \Delta E = 90 \text{ mV.}$$

EXEMPLE 4

COMPOSITION (A9)

| | |
|---|---|
| - N-butyl 5,6-dihydroxyindoline | 0,5 g |
| - Hydroxyéthylcellulose vendue sous la dénomination MATROSOL 250 HHR par la Société AQUALON | 1,0 g |
| - Alcool éthylique | 10,0 g |
| - Alkyléther de glycoside vendu sous la dénomination TRITON CG 110 par la Société ROHM & HAAS | 5,0 g MA |
| - Triéthanolamine qs | pH 5 |
| - Eau qsp | 100,0 g |

COMPOSITION (B9)

| | |
|---|---|
| - Eau oxygénée à 20 volumes | 66,0 g |
| - Solution aqueuse à 8% de monoéthanolamine | 33,0 g |

La solution (A9) est appliquée sur des cheveux à 90% de blancs pendant 10 minutes. Après rinçage, on applique la solution (B9) pendant 10 minutes. Après rinçage, lavage et séchage, les cheveux sont colorés en bleu vert.

EXEMPLE 5

COMPOSITION (A10)

Elle est similaire à la composition (A9) dans laquelle on remplace les 0,5 g de N-butyl 5,6-dihydroxyindoline par 1 g de N-méthyl 5,6-dihydroxyindoline.

COMPOSITION (B10)

Elle est identique à la composition (B9).

Les conditions de teinture sont identiques à celles décrites pour l'exemple 9.

Les cheveux sont colorés en cendré mat doré.

EXEMPLE 6

COMPOSITION (A11)

Elle est similaire à la composition (A9) dans laquelle les 0,5 g de N-butyl 5,6-dihydroxyindoline sont remplacés par 1 g de N-éthyl 5,6-dihydroxyindoline.

COMPOSITION (B11)

Elle est identique à la composition (B9).
Les conditions de teinture sont identiques à celles de l'exemple 9.
Les cheveux sont colorés en bleu vert.

EXEMPLE 7

| | |
|---|---|
| - N-méthyl 5,6-dihydroxyindoline | 0,5 g |
| - Alcool éthylique | 10,0 g |
| - Hydroxypropylcellulose vendue sous la dénomination KLUCEL G par la Société AQUALON | 2,0 g |
| - Alkyléther de glycoside vendu sous la dénomination TRITON CG 110 par la Société ROHM & HAAS | 2,1 g MA |
| - Nonylphénol oxyéthyléné à 9 moles d'oxyde d'éthylène | 0,1 g |
| - Acide tartrique | 0,3 g |
| - Triéthanolamine | 3,75 g |
| - Eau déminéralisée qsp | 100,0 g |
| - Le pH est égal à 8,5 | |

On applique cette composition sur des cheveux gris à 90% de blancs pendant 10 minutes.
Après rinçage, les cheveux sont séchés. On applique successivement deux fois cette composition, après quoi, les cheveux sont colorés en bleu gris mat.

EXEMPLE 8

| | |
|---|---|
| - N-éthyl 5,6-dihydroxyindoline | 1,0 g |
| - Alcool éthylique | 10,0 g |
| - Iodure de potassium | 1,0 g |
| - Hydroxyéthylcellulose vendue sous la dénomination NATROSOL 250 HHR par la Société AQUALON | 1,0 g |
| - Alkyléther de glycoside vendu sous la dénomination TRITON CG 110 par la Société ROHM & HAAS | 5,0 g MA |
| - Lauryléthersulfate de sodium | 0,2 g MA |
| - Triéthanolamine qs | pH 5 |
| - Eau qsp | 100,0 g |

On applique cette composition sur des cheveux gris à 90% de blancs pendant 15 minutes.
Après rinçage, on applique une solution d'eau oxygénée à 12,5 volumes pendant 5 minutes.
Après rinçage, lavage et séchage, les cheveux sont colorés en blond clair cendré.

EXEMPLE 9

Il est similaire à l'exemple 13, dans lequel on remplace les 1 g de N-éthyl 5,6-dihydroxyindoline par 1 g de N-méthyl 5,6-dihydroxyindoline.

Les conditions de teinture sont celles décrites à l'exemple 13. Les cheveux sont colorés en cendré mat doré.

EXEMPLE 10

Il est similaire à l'exemple 13, dans lequel on remplace les 1 g de N-éthyl 5,6-dihydroxyindoline par 0,5 g de N-butyl 5,6-dihydroxyindoline.

Les conditions de teinture sont celles de l'exemple 13. Les cheveux sont colorés en bleu vert.

**Revendications**

1. Utilisation pour la teinture des fibres kératiniques, en particulier des fibres kératiniques humaines, d'une 5,6-dihydroxyindoline répondant à la formule (I) :

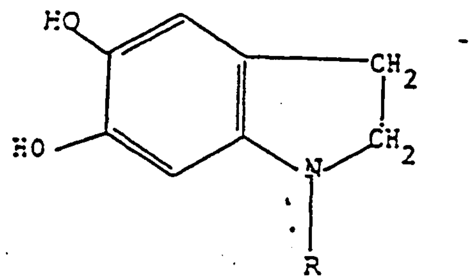

(I)

dans laquelle R représente un groupement alkyle en $C_2$-$C_4$ ainsi que les sels d'addition d'acides de ces composés.

2. Utilisation selon la revendication 1, caractérisée par le fait que les sels sont choisis parmi les chlorhydrates ou bromhydrates.

3. Utilisation de la N-éthyl 5,6-dihydroxyindoline, de la N-butyl 5,6-dihydroxyindoline, pour la teinture de fibres kératiniques.

4. Composition tinctoriale destinée à être utilisée pour la teinture de fibres kératiniques, en particulier des fibres kératiniques humaines, caractérisée par le fait qu'elle contient dans un milieu approprié pour la teinture, au moins une 5,6-dihydroxy indoline telle que définie dans l'une quelconque des revendications 1 à 3.

5. Composition selon la revendication 4, caractérisée par le fait que la 5,6-dihydroxyindoline est présente dans la composition dans des proportions comprises entre 0,01 et 8% en poids par rapport au poids total de la composition et de préférence entre 0,03 et 5% en poids.

6. Composition selon la revendication 4 ou 5, caractérisée par le fait que le milieu approprié pour la teinture est un milieu aqueux constitué par de l'eau ou un mélange eau/solvant(s).

7. Composition selon la revendication 6, caractérisée par le fait que les solvants sont choisis parmi l'alcool éthylique, l'alccol propylique ou isopropylique, l'alcool tertiobutylique, l'éthylène glycol, les éthers monométhylique, monoéthylique et monobutylique de l'éthylèneglycol, l'acétate du monoéthyléther de l'éthylèneglycol, le propylèneglycol, les monométhyléthers du propylèneglycol et du dipropylèneglycol, le lactate de méthyle.

8. Composition tinctoriale destinée à être utilisée pour la teinture des fibres kératiniques en particulier des fibres kératiniques humaines caractérisée par le fait qu'elle contient, dans un milieu approprié pour la teinture, comprenant de l'eau et au moins un solvant choisi parmi l'alcool éthylique, l'alcool propylique ou isopropylique, l'alcool tertiobutylique, l'éthylène glycol, les éthers mono méthyliques, monoéthyliques et monobutyliques de l'éthylène glycol, l'acétate du monoéthyl éther de l'éthylène glycol, le propylène glycol, les monométhyl éther du propylène glycol et du diproprylène glycol, le lactate de méthyle, au moins un composé de formule

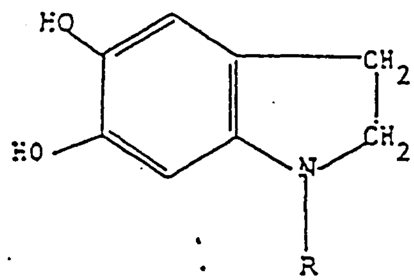

dans laquelle R désigne alkyle en $C_1$-$C_4$ ainsi que les sels d'addition d'acides de ces composés.

**9.** Composition selon l'une quelconque des revendications 4 à 8, caracterisée par le fait que la composition contient des amides gras, des agents tensioactifs anioniques, cationiques, non ioniques, amphotères ou leurs mélanges, des agents épaississants, des parfums, des agents sequestrants, des agents filmogènes, des agents de traitement, des agents dispersants, des agents de conditionnement, des agents conservateurs, des agents opacifiants, des agents de gonflement des fibres kératiniques ou leurs mélanges.

**10.** Composition selon l'une quelconque des revendications 4 à 9, caractérisée par le fait que le pH de la composition est compris entre 3 et 12.

**11.** Procédé de teinture des fibres kératiniques, en particulier des fibres kératiniques humaines, caractérisé par le fait que l'on applique sur ces fibres au moins une composition contenant dans un milieu approprié pour la teinture, au moins une 5,6-dihydroxyindoline répondant à la formule (I') :

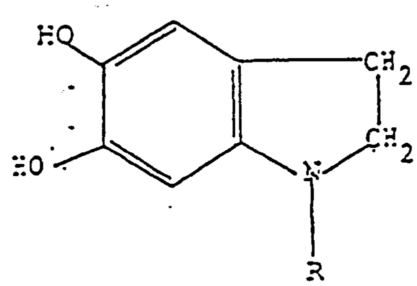

(I')

dans laquelle R représente hydrogène ou un groupement alkyle en $C_1$-$C_4$ ainsi que les sels d'addition d'acides de ces composés ; que l'on maintient cette composition au contact des fibres pendant un temps suffisant pour développer une coloration, soit à l'air, soit à l'aide d'un système oxydant chimique constitué par :

(i) des ions iodure et du peroxyde d'hydrogène, la composition (A) contenant dans ce cas, en plus, soit (a) des ions iodure, soit (b) du peroxyde d'hydrogène et l'application de la composition (A) étant précédée ou suivie par l'application d'une composition (B) qui contient dans un milieu approprié pour la teinture, soit :

(a) du peroxyde d'hydrogène à un pH compris entre 2 et 12 lorsque la composition (A) contient des ions iodure, soit :
(b) des ions iodure a un pH compris entre 3 et 11 lorsque la composition (A) contient du peroxyde d'hydrogène;

(ii) des nitrites, l'application de la composition (A) étant suivie par l'application d'une composition aqueuse (B) présentant un pH acide, la composition (A) ou la composition (B) contenant au moins un nitrite;
(iii) des oxydants choisis parmi le peroxyde d'hydrogène, l'acide périodique et ses sels hydrosolubles, l'hypochlorite de sodium, la chloramine T, la chloramine B, le ferricyanure de potassium, l'oxyde d'argent, le réactif de Fenton, l'oxyde de plomb (IV), le sulfate de cesium, le persulfate d'ammonium; ces oxydants étant présents dans la composition (A) ou appliqués simultanément ou séquentiellement au moyen d'une composition (B) les contenant dans un milieu approprié pour la teinture;

(iv) des anions d'un métal choisis parmi les permanganates ou bichromates, ces agents oxydants étant appliqués au moyen d'une composition (B) aqueuse, à un pH de 2 à 10, avant l'application de la composition (A);

(v) des sels métalliques des groupes 3 à 8 du tableau périodique, ces sels métalliques étant appliqués dans une étape séparée au moyen d'une composition (B) contenant ces sels dans un milieu approprié pour la teinture;

(vi) des sels de terres rares, ces sels de terres rares étant appliqués au moyen d'une composition contenant ces sels dans un milieu approprié pour la teinture, la composition (B) étant appliquée préalablement ou postérieurement à l'application de la composition (A);

(vii) un dérivé quinonique choisi parmi les ortho- ou parabenzoquinones, les ortho- ou parabenzoquinones monoïmines ou diimines, les 1,2- ou 1,4-naphtoquinones, les ortho- ou parabenzoquinones sulfonimides, les α, ω-alkylène-bis-1,4-benzoquinones ou les 1,2-ou 1,4-naphtoquinones monoimines ou diimines, la 5,6-dihydroxyindoline de formule (I) et les dérivés quinoniques étant choisis de façon à ce que la différence de potentiel d'oxydoréduction ΔE entre le potentiel d'oxydoréduction Ei de la 5,6-di-hydroxy indoline de formule (I) déterminé à pH 7 en milieu phosphate sur électrode au carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leqq 320 \text{ millivolts};$$

la composition (B) étant appliquée préalablement ou postérieurement à l'application de la composition (A); sous réserve que lorsque le composé de formule (I') est la 5,6-dihydroxyindoline ou la N-méthyl 5,6-dihydroxindoline ou l'un de leurs sels d'addition d'acides, le système oxydant est différent de l'air, du péroxyde d'hydrogène, de l'acide périodique et ses sels hydrosolubles, du persulfate d'ammonium ou de potassium.

**12.** Procédé de teinture des fibres kératiniques, en particulier des fibre kératiniques humaines, caractérisé par le fait que l'on applique sur ces fibres au moins une composition contenant dans un milieu approprié pour la teinture comprenant de l'eau et au moins un solvant ou uniquement un ou plusieurs solvants anhydres, ces solvants étant choisis parmi l'alcool éthylique, l'alcool propylique ou isopropylique, l'alcool tertiobutylique, l'éthylène glycol, les éthers mono méthyliques, monoéthyliques et monobutyliques de l'éthylène glycol, l'acétate du monoéthyl éther de l'éthylène glycol, le propylène glycol,les monométhyl éther du propylène glycol et du dipropylène glycol, le lactate de méthyle au moins une 5,6-dihydroxyindoline répondant à la formule (I')

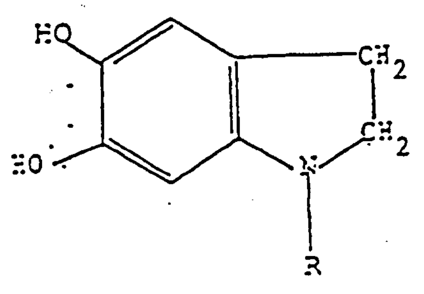

(I')

dans laquelle R représente hydrogène ou un groupement alkyle en $C_1$-$C_4$ ainsi que les sels d'addition d'acides de ces composés ; que l'on maintient cette composition au contact des fibres pendant un temps suffisant pour développer une coloration, soit à l'air, soit à l'aide d'un système oxydant chimique constitué par:

(i) des ions iodure et du peroxyde d'hydrogène, la composition (A) contenant dans ce cas, en plus, soit (a) des ions iodure, soit (b) du peroxyde d'hydrogène et l'application de la composition (A) étant précédée ou suivie par l'application d'une composition (B) qui contient dans un milieu approprié pour la teinture, soit :

(a) du peroxyde d'hydrogène à un pH compris entre 2 et 12 lorsque la composition (A) contient des ions iodure, soit :

(b) des ions iodure a un pH compris entre 3 et 11 lorsque la composition (A) contient du peroxyde d'hydrogène;

(ii) des nitrites, l'application de la composition (A) étant suivie par l'application d'une composition aqueuse (B) présentant un pH acide, la composition (A) ou la composition (B) contenant au moins un nitrite;

(iii) des oxydants choisis parmi le peroxyde d'hydrogène, l'acide périodique et ses sels hydrosolubles. l'hypochlorite de sodium, la chloramine T, la chloramine B, le ferricyanure de potassium, l'oxyde d'argent, le réactif de Fenton, l'oxyde de plomb (IV), le sulfate de cesium, le persulfate d'ammonium; ces oxydants étant présents dans la composition (A) ou appliqués simultanément ou séquentiellement au moyen d'une composition (B) les contenant dans un milieu approprié pour la teinture;

(iv) des anions d'un métal choisis parmi les permanganates ou bichromates, ces agents oxydants étant appliqués au moyen d'une composition (B) aqueuse, à un pH de 2 à 10, avant l'application de la composition (A);

(v) des sels métalliques des groupes 3 à 8 du tableau périodique, ces sels métalliques étant appliqués dans une étape séparée au moyen d'une composition (B) contenant ces sels dans un milieu approprié pour la teinture;

(vi) des sels de terres rares, ces sels de terres rares étant appliqués au moyen d'une composition contenant ces sels dans un milieu approprié pour la teinture, la composition (B) étant appliquée préalablement ou postérieurement à l'application de la composition (A);

(vii) un dérivé quinonique choisi parmi les ortho- ou parabenzoquinones, les ortho- ou parabenzoquinones monoïmines ou diimines, les 1,2-ou 1,4-naphtoquinones, les ortho- ou parabenzoquinones sulfonimides, les $\alpha$, $\omega$-alkylène-bis-1,4-benzoquinones ou les 1,2-ou 1,4-naphtoquinones monoimines ou diimines, la 5,6-dihydroxyindoline de formule (I) et les dérivés quinoniques étant choisis de façon à ce que la différence de potentiel d'oxydoréduction $\Delta E$ entre le potentiel d'oxydoréduction Ei de la 5,6-dihydroxy indoline de formule (I) déterminé à pH 7 en milieu phosphate sur électrode au carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leqq 320 \text{ millivolts};$$

la composition (B) étant appliquée préalablement ou postérieurement à l'application de la composition (A)

**13.** Procédé selon la revendication 11 ou 12, caractérisé par le fait que l'on laisse la coloration se développer au contact de l'air sans additionner d'agent oxydant extérieur.

**14.** Procédé selon la revendication 11 ou 12, caractérisé par le fait que l'on applique sur les matières kératiniques une composition (A) contenant dans un milieu approprié pour la teinture, au moins une 5,6-dihydroxyindoline de formule (I') en association avec des ions iodure, l'application de la composition (A) étant précédée ou suivie par l'application d'une composition (B) qui contient dans un milieu approprié pour la teinture, du peroxyde d'hydrogène.

**15.** Procédé selon la revendication 11 ou 12, caractérisé par le fait que l'on applique sur les fibres kératiniques au moins une composition (A) contenant dans un milieu approprié pour la teinture, une 5,6-dihydroxyindoline de formule (I') en association avec du peroxyde d'hydrogène et ayant un pH compris entre 2 et 7, l'application de la composition (A) étant précédée ou suivie par l'application d'une composition (B) qui contient dans un milieu approprié pour la teinture, des ions iodure.

**16.** Procédé selon la revendication 14 ou 15, caractérisé par le fait que les ions iodure sont présents dans la composition (A) ou (B) dans des proportions comprises entre 0,007 et 4% en poids exprimées en ions $I^-$ par rapport au poids total de la composition (A) ou (B).

**17.** Procédé selon la revendication 11 ou 12, caractérisé par le fait que l'on applique sur les matières kératiniques une composition (A) contenant la 5,6-dihydroxyindoline de formule (I') et que l'on applique ensuite une composition aqueuse acide (B), la composition (A) ou la composition (B) contenant au moins un nitrite choisi parmi les nitrites de métaux alcalins, alcalino-terreux ou d'ammonium ou de tout autre cation cosmétiquement acceptable, un dérivé organique de nitrite et des vecteurs de nitrite générant un nitrite du type défini ci-dessus.

**18.** Procédé selon la revendication 17 caractérisé par le fait que les nitrites sont présents dans des proportions comprises entre 0,02 et 1 mole/litre.

**19.** Procédé selon la revendication 11 ou 12, caractérisé par le fait que l'on applique sur les fibres kératiniques une composition (B) contenant dans un milieu approprié pour la teinture, à un pH compris entre 2 et 10, un anion d'un métal ayant une bonne affinité pour la kératine choisi parmi les permanganates ou les bichromates et que dans un second temps, on applique une composition (A) contenant dans un milieu approprié pour la teinture, à un pH compris entre 4 et 10, une 5,6-dihydroxyindoline répondant à la formule (I').

**20.** Procédé selon la revendication 19, caractérisé par le fait que les permanganates ou bichromates sont utilisés dans des molalités en anions supérieures à $10^{-3}$ moles/1000 g jusqu'à de préférence 1 mole/1000 g et que les compositions ne contiennent pas d'agent organique ayant un effet réducteur sur les anions.

**21.** Procédé selon la revendication 11 ou 12, caractérisé par le fait que l'on applique sur les fibres kératiniques une composition (A) contenant dans un milieu approprié pour la teinture, une 5,6-dihydroxy indoline de formule (I') et que l'on applique avant ou après la composition (A) une composition (B) contenant dans un milieu approprié pour la teinture, un sel métallique choisi parmi les sels de manganèse, de cobalt, de fer, de cuivre et d'argent.

**22.** Procédé selon la revendication, 21, caractérisé par le fait que les sels de métaux sont utilisés dans des proportions comprises entre 0,01 et 2% en poids exprimé en ions métalliques par rapport au poids total de la composition.

**23.** Procédé selon la revendication 11 ou 12, caractérisé par le fait que l'on applique une composition (A) contenant dans un milieu approprié pour la teinture, au moins une 5,6-dihydroxyindoline de formule (I') et qu'avant ou après cette composition, on applique une composition (B) contenant dans un milieu approprié pour la teinture, un sel de terres rares choisi parmi les-sels de Cériun, de Lanthane, d'Europium, de Gadolinium, d'Ytterbium, de Dysprosium.

**24.** Procédé selon la revendication 23, caractérisé par le fait que les sels de terres rares sont présents dans des proportions comprises entre 0,1 et 8% en poids par rapport au poids total de la composition.

**25.** Procédé selon la revendication 11 ou 12, caractérisé par le fait que lorsqu'on utilise comme moyen oxydant une composition à base de peroxyde d'hydrogène, la teneur en peroxyde d'hydrogène dans la composition est comprise entre 1 et 40 volumes, de préférence entre 2 et 10 volumes.

**26.** Agent de teinture des fibres kératiniques, en particulier des fibres kératiniques humaines, à plusieurs composants, caractérisé par le fait qu'il comprend un premier composant constitué par une composition (A) contenant une 5,6-dihydroxyindoline de formule (I') telle que définie dans la revendication 11 et un second composant constitué par l'une des compositions (B) définie dans l'une quelconque des revendications 11 à 25, sous reserve que lorsque le compose de formule I' est la 5,6-dihydroxyindoline ou la N-methyl 5,6-dihydroxyindoline ou l'un de leurs sels d'addition d'acides, le système oxydant est différent de l'air, du peroxyde d'hydrogène, de l'acide periodique et ses sels hydrosolubles, du persulfate d'ammonium ou potassium.

**27.** Agent de teinture des fibres kératiniques, en particulier des fibres kératiniques humaines, à plusieurs composants, caractérisé par le fait qu'il comprend un premier composant constitué par une composition (A) contenant une 5,6-dihydroxyindoline répondant à la formule (I') :

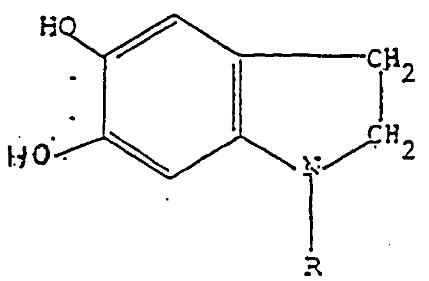

(I')

dans laquelle R représente hydrogène ou un groupement alkyle en $C_1$-$C_4$ ainsi que les sels d'addition d'acides de ces composés dans un milieu comprenant de l'eau et au moins un solvant ou uniquement un ou plusieurs solvants anhydres, ces solvants étant choisis parmi l'alcool éthylique, l'alcool propylique ou isopropylique, l'alcool tertiobutylique, l'éthylène glycol, les éthers mono méthyliques, monoéthyliques et mono- butyliques de l'éthylène glycol, l'acétate du monoéthyl éther de l'éthylène glycol, le propylène glycol,les monométhyl éther du propylène glycol et du dipropylène glycol, le lactate de méthyle et un second composant constitué par un système oxydant chimique choisi parmi :

(i) une composition (B) qui contient dans un milieu approprié pour la teinture, soit :

a) du peroxyde d'hydrogène à un pH compris entre 2 et 12 lorsque la composition (A) contient des ions

iodure, soit;

b) des ions iodure a un pH compris entre 3 et 11 lorsque la composition (A) contient du peroxyde d'hydrogène;

(ii) une composition aqueuse (B) présentant un pH acide, lorsque la composition (A) contient un nitrite ou une composition (B) aqueuse à pH acide contenant au moins un nitrite;
(iii) une composition (B) contenant un oxydant choisi parmi le peroxyde d'hydrogène, l'acide périodique et ses sels hydrosolubles, l'hypochlorite de sodium, la chloramine T, la chloramine B, le ferricyanure de potassium, l'oxyde d'argent, le réactif de Fenton, l'oxyde plomb (IV), le sulfate de cesium, le persulfate d'ammonium dans un milieu approprié pour la teinture :
(iv) une composition (B) aqueuse contenant des anions d'un métal choisis parmi les permanganate ou bichromate àun pH de 2 à 10,
(v) une composition (B) contenant des sels métalliques des groupes III à VIII du tableau périodique dans un milieu approprié pour la teinture :
(vi) une composition (B) contenant des sels de terres rares, dans un milieu approprié pour la teinture :
(vii) une composition (B) contenant un dérivé quinonique choisi parmi les ortho- ou parabenzoquinones, les ortho- ou parabenzoquinones monoïmines ou diimines, les 1,2- ou 1,4-naphtoquinones, les ortho- ou parabenzoquinones sulfonimides, les $\alpha,\omega$ -alkylène-bis- 1,4-benzoquinones ou les 1,2- ou 1,4-naphtoquinones monoïmines, la 5,6-dihydroxyindoline de formule (I)' de la composition (A) et les dérivés quinoniques de la composition (B) étant choisis de façon à ce que la différence de potentiel d'oxydoréduction $\Delta E$ entre le potentiel d'oxydoréduction Ei de la 5,6-dihydroxyindoline de formule (I') déterminé à pH 7 en milieu phosphate sur électrode au carbone vitreux par voltamétrie et le potentiel d'oxydoréduction Eq du dérivé quinonique déterminé à pH 7 en milieu phosphate par polarographie sur électrode de mercure par rapport à l'électrode au calomel saturé, soit telle que :

$$\Delta E = Ei - Eq \leq 320 \text{ millivolts}$$

**28.** Agent selon la revendication 27, caractérisé par le fait que la composition (A) contient dans un milieu approprié pour la teinture au moins une 5,6-dihydroxyindoline de formule (I') en association avec des ions iodure, la composition (B) contient dans un milieu approprié pour la teinture, du peroxyde d'hydrogène.

**29.** Agent selon la revendication 27, caractérisé par le fait que la composition (A) contient dans un milieu approprié pour la teinture, au moins une 5,6-dihydroxyindoline de formule (I') en association avec du peroxyde d'hydrogène et a un pH compris entre 2 et 7, la composition (B) contient dans un milieu approprié pour la teinture, des ions iodure.

**30.** Agent selon la revendication 28 ou 29, caractérisé par le fait que les ions iodure sont présents dans la composition (A) ou (B) dans des proportions comprises entre 0,007 et 4% en poids exprimées en ions $I^-$par rapport au poids total de la composition (A) ou (B).

**31.** Agent selon la revendication 27, caractérisé par le fait que la composition (A) contient la 5,6-dihydroxyindoline de formule (I') et que la composition (B) est aqueuse acide, la composition (A) ou la composition (B) contenant au moins un nitrite choisi parmi les les nitrites de métaux alcalins, alcalinoterreux ou d'ammonium ou de tout autre cation cosmétiquement acceptable, un dérivé organique de nitrite et des vecteurs de nitrite générant un nitrite du type défini ci-dessus.

**32.** Agent selon la revendication 31, caractérisé par le fait que les nitrites sont présents dans des proportions comprises entre 0,02 et 1 mole/litre.

**33.** Agent selon la revendication 27, caractérisé par le fait que la composition (B) contient dans un milieu approprié pour la teinture, à un pH compris entre 2 et 10, un anion d'un métal ayant une bonne affinité pour la kératine choisi parmi les permanganates ou les bichromates.

**34.** Agent selon la revendication 33, caractérisé par le fait que les permanganates ou les bichromates sont utilisés dans des molalités en anions supérieures à $10^{-3}$ moles/1000g jusqu'à de préférence 1 mole/1000g et que les compositions ne contiennent pas d'agent organique ayant un effet réducteur sur les anions.

**35.** Agent selon la revendication 27, caractérisé par le fait que la composition (B) contient dans un milieu approprié

pour la teinture, un sel métallique choisi parmi les sels de manganèse, de cobalt, de fer, de cuivre et d'argent.

**36.** Agent selon la revendication 35, caractérisé par le fait que les sels de métaux sont utilisés dans des proportions comprises entre 0,01 et 2% en poids exprimé en ions métallliques par rapport au poids toal de la composition.

**37.** Agent selon la revendication 27, caractérisé par le fait que la composition (B) contient dans un milieu approprié pour la teinture, un sel de terres rares choisi parmi les sels de Cérium, de Lanthane, d'Europium, de Gadolinium, d'Ytterbium, de Dysprosium.

**38.** Agent selon la revendication 37, caractérisé par le fait que les sels de terres rares sont présents dans des proportions comprises entre 0,1 et 8% en poids par rapport au poids total de la composition.

**39.** Agent selon la revendication 27, caractérisé par le fait que la composition (B) est à base de peroxyde d'hydrogène, la teneur en peroxyde d'hydrogène dans la composition est comprise entre 1 et 40 volumes, de préférence entre 2 et 10 volumes.

**40.** Dispositif à plusieurs compartiments ou "kit de teinture", caractérisé par le fait que ces différents compartiments contiennent les différents composants de l'agent de teinture défini dans l'une quelconque des revendications 26 à 39.

## Claims

**1.** Use of a 5,6-dihydroxyindoline corresponding to the formula (I):

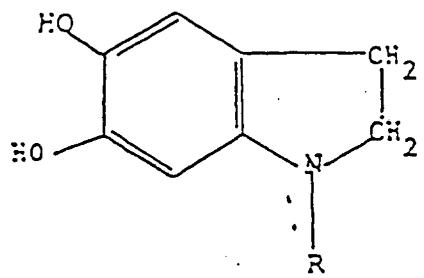

(I)

in which R represents a $C_2$-$C_4$ alkyl group, as well as the acid addition salts of these compounds for dyeing keratinous fibres, in particular human keratinous fibres.

**2.** Use according to Claim 1, characterised in that the salts are chosen from the hydrochlorides or hydrobromides.

**3.** Use of N-ethyl-5,6-dihydroxyindoline and N-butyl-5,6-dihydroxyindoline for dyeing keratinous fibres.

**4.** Tinctorial composition intended to be used for dyeing keratinous fibres, in particular human keratinous fibres, characterised in that it contains at least one 5,6-dihydroxyindoline as defined in any one of Claims 1 to 3 in a medium appropriate for dyeing.

**5.** Composition according to Claim 4, characterised in that the 5,6-dihydroxyindoline is present in the composition in proportions of between 0.01 and 8% by weight relative to the total weight of the composition, and preferably of between 0.03 and 5% by weight.

**6.** Composition according to Claim 4 or 5, characterised in that the medium appropriate for dyeing is an aqueous medium consisting of water or a water/solvent(s) mixture.

**7.** Composition according to Claim 6, characterised in that the solvents are chosen from ethyl alcohol, propyl alcohol or isopropyl alcohol, tert-butyl alcohol, ethylene glycol, ethylene glycol monomethyl, monoethyl and monobutyl ethers, ethylene glycol monoethyl ether acetate, propylene glycol, propylene glycol and dipropylene glycol monomethyl ethers and methyl lactate.

8. Tinctorial composition intended to be used for dyeing keratinous fibres, in particular human keratinous fibres, characterised in that it contains, in a medium appropriate for dyeing, comprising water and at least one solvent chosen from ethyl alcohol, propyl or isopropyl alcohol, tert-butyl alcohol, ethylene glycol, ethylene glycol monomethyl, monoethyl and monobutyl ethers, ethylene glycol monoethyl ether acetate, propylene glycol, propylene glycol and dipropylene glycol monomethyl ether and methyl lactate, at least one compound of formula

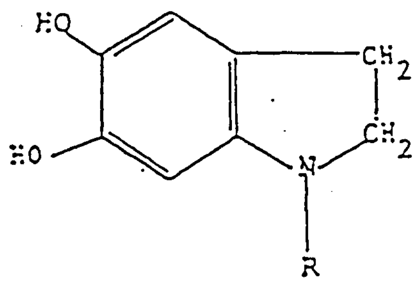

in which R designates a $C_1$-$C_4$ alkyl, as well as the acid addition salts of these compounds.

9. Composition according to any one of Claims 4 to 8, characterised in that the composition contains fatty amides, anionic, cationic, nonionic or amphoteric surfactants, or their mixtures, thickeners, perfumes, sequestering agents, film-forming agents, treatment agents, dispersing agents, conditioners, preservatives, opacifying agents and agents for swelling keratinous fibres or their mixtures.

10. Composition according to any one of Claims 4 to 9, characterised in that the pH of the composition is between 3 and 12.

11. Method for dyeing keratinous fibres, in particular human keratinous fibres, characterised in that at least one composition containing, in a medium appropriate for dyeing, at least one 5,6-dihydroxyindoline corresponding to the formula (I'):

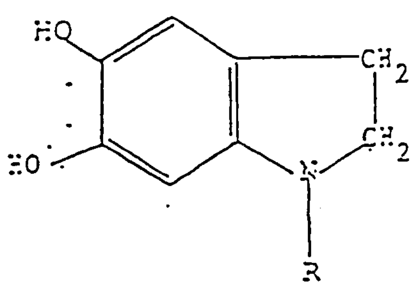

(I')

in which R represents hydrogen or a $C_1$-$C_4$ alkyl group, as well as the acid addition salts of these compounds is applied to these fibres, in that this composition is kept in contact with the fibres for a period sufficient to develop a colour, either in the air or with the aid of a chemical oxidising system consisting of:

(i) iodide ions and hydrogen peroxide, the composition (A) additionally containing, in this case, either (a) iodide ions or (b) hydrogen peroxide and the application of the composition (A) being preceded or followed by the application of a composition (B) which contains, in a medium appropriate for dyeing, either:

(a) hydrogen peroxide at a pH of between 2 and 12 when the composition (A) contains iodide ions, or:
(b) iodide ions at a pH of between 3 and 11, when the composition (A) contains hydrogen peroxide;

(ii) nitrites, the application of the composition (A) being followed by the application of an aqueous composition (B) having an acid pH, the composition (A) or the composition (B) containing at least one nitrite;
(iii) oxidants chosen from hydrogen peroxide, periodic acid and its water-soluble salts, sodium hypochlorite, chloramine T, chloramine B, potassium ferricyanide, silver oxide, Fenton's reagent, lead(IV) oxide, caesium sulphate and ammonium persulphate; these oxidants being present in the composition (A) or being applied simultaneously or sequentially by means of a composition (B) containing them in a medium appropriate for dyeing;

(iv) metal anions chosen from permanganates or dichromates, these oxidising agents being applied by means of an aqueous composition (B), at a pH of 2 to 10, before the application of the composition (A);

(v) salts of metals of groups 3 to 8 of the periodic table, these metal salts being applied in a separate step by means of a composition (B) containing these salts in a medium appropriate for dyeing;

(vi) rare-earth salts, these rare-earth salts being applied by means of a composition containing these salts in a medium appropriate for dyeing, the composition (B) being applied before or after the application of the composition (A); and

(vii) a quinone derivative chosen from ortho- or para-benzoquinones, ortho- or para-benzoquinone monoimines or diimines, 1,2- or 1,4-naphthoquinones, ortho- or para-benzoquinone sulphonimides, α,ω-alkylene-bis-1,4-benzoquinones or 1,2- or 1,4-naphthoquinone monoimines or diimines, the 5,6-dihydroxyindoline of formula (I) and the quinone derivatives being chosen such that the difference in redox potential ΔE between the redox potential $E_i$ of the 5,6-dihydroxyindoline of formula (I) determined at pH 7 in a phosphate medium on a vitreous carbon electrode by means of voltammetry and the redox potential $E_q$ of the quinone derivative determined at pH 7 in a phosphate medium by polarography on a mercury electrode relative to the saturated calomel electrode is such that:

$$\Delta E = E_i - E_q \leq 320 \text{ millivolts;}$$

the composition (B) being applied before or after the application of the composition (A); with the proviso that when the compound of formula (I') is 5,6-dihydroxyindoline or N-methyl-5,6-dihydroxyindoline or one of their acid addition salts, the oxidising system is different from air, hydrogen peroxide, periodic acid and its water-soluble salts, and ammonium or potassium persulphate.

**12.** Method for dyeing keratinous fibres, in particular human keratinous fibres, characterised in that there is applied to these fibres at least one composition containing, in a medium appropriate for dyeing comprising water and at least one solvent or only one or more anhydrous solvents, these solvents being chosen from ethyl alcohol, propyl or isopropyl alcohol, tert-butyl alcohol, ethylene glycol, ethylene glycol monomethyl, monoethyl and monobutyl ethers, ethylene glycol monoethyl ether acetate, propylene glycol, propylene glycol and dipropylene glycol monomethyl ether and methyl lactate, at least one 5,6-dihydroxyindoline corresponding to the formula (I')

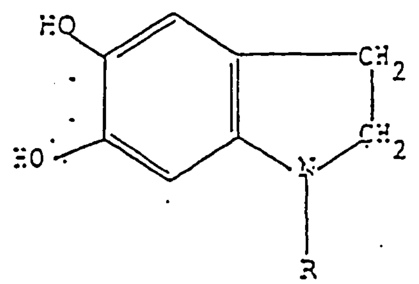

(I')

in which R represents hydrogen or a $C_1$-$C_4$ alkyl group, as well as the acid addition salts of these compounds; in that this composition is kept in contact with the fibres for a period sufficient to develop a colour, either in the air or with the aid of a chemical oxidising system consisting of:

(i) iodide ions and hydrogen peroxide, the composition (A) additionally containing, in this case, either (a) iodide ions or (b) hydrogen peroxide and the application of the composition (A) being preceded or followed by the application of a composition (B) which contains, in a medium appropriate for dyeing, either:

(a) hydrogen peroxide at a pH of between 2 and 12 when the composition (A) contains iodide ions, or:
(b) iodide ions at a pH of between 3 and 11, when the composition (A) contains hydrogen peroxide;

(ii) nitrites, the application of the composition (A) being followed by the application of an aqueous composition (B) having an acid pH, the composition (A) or the composition (B) containing at least one nitrite;

(iii) oxidants chosen from hydrogen peroxide, periodic acid and its water-soluble salts, sodium hypochlorite, chloramine T, chloramine B, potassium ferricyanide, silver oxide, Fenton's reagent, lead(IV) oxide, caesium sulphate and ammonium persulphate; these oxidants being present in the composition (A) or being applied simultaneously or sequentially by means of a composition (B) containing them in a medium appropriate for dyeing;
(iv) metal anions chosen from permanganates or dichromates, these oxidising agents being applied by means of an aqueous composition (B), at a pH of 2 to 10, before the application of the composition (A);
(v) salts of metals of groups 3 to 8 of the periodic table, these metal salts being applied in a separate step by means of a composition (B) containing these salts in a medium appropriate for dyeing;
(vi) rare-earth salts, these rare-earth salts being applied by means of a composition containing these salts in a medium appropriate for dyeing, the composition (B) being applied before or after the application of the composition (A); and
(vii) a quinone derivative chosen from ortho- or para-benzoquinones, ortho- or para-benzoquinone monoimines or diimines, 1,2- or 1,4-naphthoquinones, ortho- or para-benzoquinone sulphonimides, $\alpha,\omega$-alkylene-bis-1,4-benzoquinones or 1,2- or 1,4-naphthoquinone monoimines or diimines, the 5,6-dihydroxyindoline of formula (I) and the quinone derivatives being chosen such that the difference in redox potential $\Delta E$ between the redox potential $E_i$ of the 5,6-dihydroxyindoline of formula (I) determined at pH 7 in a phosphate medium on a vitreous carbon electrode by means of voltammetry and the redox potential $E_q$ of the quinone derivative determined at pH 7 in a phosphate medium by polarography on a mercury electrode relative to the saturated calomel electrode is such that:

$$\Delta E = E_i - E_q \leq 320 \text{ millivolts;}$$

the composition (B) being applied before or after the application of the composition (A)

**13.** Method according to Claim 11 or 12, characterised in that the colour is allowed to develop in contact with the air without adding external oxidising agent.

**14.** Method according to Claim 11 or 12, characterised in that a composition (A) containing, in a medium appropriate for dyeing, at least one 5,6-dihydroxyindoline of formula (I') in combination with iodide ions is applied to the keratinous materials, the application of the composition (A) being preceded or followed by the application of a composition (B) which contains hydrogen peroxide in a medium appropriate for dyeing.

**15.** Method according to Claim 11 or 12, characterised in that at least one composition (A) containing, in a medium appropriate for dyeing, a 5,6-dihydroxyindoline of formula (I') in combination with hydrogen peroxide and having a pH of between 2 and 7 is applied to the keratinous fibres, the application of the composition (A) being preceded or followed by the application of a composition (B) which contains iodide ions in a medium appropriate for dyeing.

**16.** Method according to Claim 14 or 15, characterised in that the iodide ions are present in the composition (A) or (B) in proportions of between 0.007 and 4% by weight, expressed as $I^-$ ions, relative to the total weight of the composition (A) or (B).

**17.** Method according to Claim 11 or 12, characterised in that a composition (A) containing the 5,6-dihydroxyindoline of formula (I') is applied to the keratinous materials and in that an acid aqueous composition (B) is then applied, the composition (A) or the composition (B) containing at least one nitrite chosen from alkali metal nitrites, alkaline-earth metal nitrites or ammonium nitrite or the nitrite of any other cosmetically acceptable cation, an organic nitrite derivative and nitrite vectors generating a nitrite of the type defined above.

**18.** Method according to Claim 17, characterised in that the nitrites are present in proportions of between 0.02 and 1 mole/litre.

**19.** Method according to Claim 11 or 12, characterised in that a composition (B) containing, in a medium appropriate for dyeing, at a pH of between 2 and 10, a metal anion having a good affinity for keratin, chosen from permanganates or dichromates is applied to the keratinous fibres and in that, in a second step, a composition (A) containing a 5,6-dihydroxyindoline corresponding to the formula (I') in a medium appropriate for dyeing, at a pH of between 4 and 10, is applied.

**20.** Method according to Claim 19, characterised in that the permanganates or dichromates are used in anion molalities of higher than $10^{-3}$ moles/1,000 g up to preferably 1 mole/1,000 g and in that the compositions do not contain an organic agent having a reducing effect on the anions.

21. Method according to Claim 11 or 12, characterised in that a composition (A) containing a 5,6-dihydroxyindoline of formula (I') in a medium appropriate for dyeing is applied to the keratinous fibres and in that a composition (B) containing, in a medium appropriate for dyeing, a metal salt chosen from the manganese, cobalt, iron, copper and silver salts is applied before or after the composition (A).

22. Method according to Claim 21, characterised in that the metal salts are used in proportions of between 0.01 and 2% by weight, expressed as metal ions, relative to the total weight of the composition.

23. Method according to Claim 11 or 12, characterised in that a composition (A) containing at least one 5,6-dihydroxyindoline of formula (I') in a medium appropriate for dyeing is applied and in that, before or after this composition, a composition (B) containing, in a medium appropriate for dyeing, a rare-earth salt chosen from the cerium, lanthanum, europium, gadolinium, ytterbium and dysprosium salts is applied.

24. Method according to Claim 23, characterised in that the rare-earth salts are present in proportions of between 0.1 and 8% by weight relative to the total weight of the composition.

25. Method according to Claim 11 or 12, characterised in that when a composition based on hydrogen peroxide is used as the oxidising medium, the hydrogen peroxide content in the composition is between 1 and 40 volumes, preferably between 2 and 10 volumes.

26. Multicomponent agent for dyeing keratinous fibres, in particular human keratinous fibres, characterised in that it comprises a first component consisting of a composition (A) containing a 5,6-dihydroxyindoline of formula (I') as defined in Claim 11, and a second component consisting of one of the compositions (B) defined in any one of Claims 11 to 25, with the proviso that when the compound of formula (I') is 5,6-dihydroxyindoline or N-methyl-5,6-dihydroxyindoline or one of their acid addition salts, the oxidising system is different from air, hydrogen peroxide, periodic acid and its water-soluble salts, ammonium or potassium persulphate.

27. Multicomponent agent for dyeing keratinous fibres, in particular human keratinous fibres, characterised in that it comprises a first component consisting of a composition (A) containing a 5,6-dihydroxyindoline corresponding to the formula (I'):

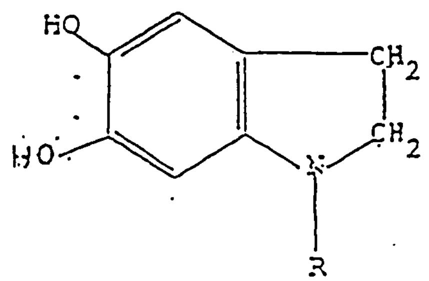

(I')

in which R represents hydrogen or a $C_1$-$C_4$ alkyl group, as well as the acid addition salts of these compounds, in a medium comprising water and at least one solvent or only one or more anhydrous solvents, these solvents being chosen from ethyl alcohol, propyl or isopropyl alcohol, tert-butyl alcohol, ethylene glycol, ethylene glycol monomethyl, monoethyl and monobutyl ethers, ethylene glycol monoethyl ether acetate, propylene glycol, propylene glycol and dipropylene glycol monomethyl ether, methyl lactate and a second component consisting of a chemical oxidising system chosen from:

(i) a composition (B) which contains, in a medium appropriate for dyeing, either:

(a) hydrogen peroxide at a pH of between 2 and 12, when the composition (A) contains iodide ions, or:
(b) iodide ions at a pH of between 3 and 11 when the composition (A) contains hydrogen peroxide;

(ii) an aqueous composition (B) having an acidic pH, when the composition (A) contains a nitrite or an aqueous composition (B) at acidic pH containing at least one nitrite;
(iii) a composition (B) containing an oxidant chosen from hydrogen peroxide, periodic acid and its water-soluble salts, sodium hypochlorite, chloramine T, chloramine B, potassium ferricyanide, silver oxide, Fenton's reagent,

lead(IV) oxide, caesium sulphate and ammonium persulphate in a medium appropriate for dyeing:

(iv) an aqueous composition (B) containing metal anions chosen from permanganate or dichromate at a pH of 2 to 10,

(v) a composition (B) containing metal salts of groups III to VIII of the periodic table, in a medium appropriate for dyeing:

(vi) a composition (B) containing rare-earth salts, in a medium appropriate for dyeing:

(vii) a composition (B) containing a quinone derivative chosen from the ortho- or para-benzoquinones, the ortho- or para-benzoquinone monoimines or diimines, the 1,2- or 1,4-naphthoquinones, the ortho- or parabenzoquinone sulphonimides, $\alpha,\omega$-alkylene-bis-1,4-benzoquinones or the 1,2- or 1,4-naphthoquinone monoimines, 5,6-dihydroxyindole of formula (I') of the composition (A) and the quinone derivatives of the composition (B) being chosen so that the difference in redox potential $\Delta E$ between the redox potential $E_i$ of the 5,6-dihydroxyindoline of formula (I') determined at pH 7 in phosphate medium on a vitreous carbon electrode by voltammetry and the redox potential $E_q$ of the quinone derivative determined at pH 7 in phosphate medium by polarography on a mercury electrode relative to the saturated calomel electrode, is such that:

$$\Delta E = E_i - E_q \leq 320 \text{ millivolts.}$$

**28.** Agent according to Claim 27, characterised in that the composition (A) contains, in a medium appropriate for dyeing, at least one 5,6-dihydroxyindoline of formula (I') in combination with iodide ions, the composition (B) contains, in a medium appropriate for dyeing, hydrogen peroxide.

**29.** Agent according to Claim 27, characterised in that the composition (A) contains, in a medium appropriate for dyeing, at least one 5,6-hydroxyindoline of formula (I') in combination with hydrogen peroxide and has a pH of between 2 and 7, the composition (B) contains, in a medium appropriate for dyeing, iodide ions.

**30.** Agent according to Claim 28 or 29, characterised in that the iodide ions are present in the composition (A) or (B) in proportions of between 0.007 and 4% by weight, expressed in $I^-$ ions, relative to the total weight of the composition (A) or (B).

**31.** Agent according to Claim 27, characterised in that the composition (A) contains 5,6-dihydroxyindoline of formula (I') and in that the composition (B) is aqueous acidic, the composition (A) or the composition (B) containing at least one nitrite chosen from alkali metal nitrites, alkaline-earth metal nitrites or ammonium nitrite or the nitrite of any other cosmetically acceptable cation, an organic nitrite derivative and nitrite vectors generating a nitrite of the type defined above.

**32.** Agent according to Claim 31, characterised in that the nitrites are present in proportions of between 0.02 and 1 mole/litre.

**33.** Agent according to Claim 27, characterised in that the composition (B) contains, in a medium appropriate for dyeing, at a pH of between 2 and 10, a metal anion having good affinity for keratin, chosen from permanganates or dichromates.

**34.** Agent according to Claim 33, characterised in that the permanganates or dichromates are used in anion molalities of higher than $10^{-3}$ moles/1000 g up to preferably 1 mole/1000 g, and in that the compositions do not contain an organic agent having a reducing effect on the anions.

**35.** Agent according to Claim 27, characterised in that the composition (B) contains, in a medium appropriate for dyeing, a metal salt chosen from manganese, cobalt, iron, copper and silver salts.

**36.** Agent according to Claim 35, characterised in that the metal salts are used in proportions of between 0.01 and 2% by weight, expressed as metal ions, relative to the total weight of the composition.

**37.** Agent according to Claim 27, characterised in that the composition (B) contains, in a medium appropriate for dyeing, a rare-earth salt chosen from cerium, lanthanum, europium, gadolinium, ytterbium and dysprosium salts.

**38.** Agent according to Claim 37, characterised in that the rare-earth salts are present in proportions of between 0.1 and 8% by weight relative to the total weight of the composition.

39. Agent according to Claim 27, characterised in that the composition (B) is based on hydrogen peroxide, the hydrogen peroxide contained in the composition is between 1 and 40 volumes, preferably between 2 and 10 volumes.

40. Multicompartment device or "dyeing kit", characterised in that these different compartments contain different components of the dyeing agent defined in any one of Claims 26 to 39.

**Patentansprüche**

1. Verwendung eines 5,6-Dihydroxyindolins der Formel (I):

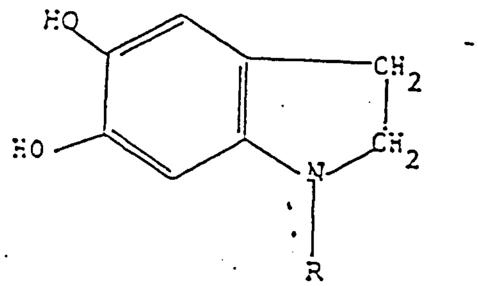

(I)

worin R eine $C_{2-4}$-Alkylgruppe darstellt, sowie der Säureadditionssalze dieser Verbindungen zur Färbung keratinischer Fasern, insbesondere menschlicher keratinischer Fasern.

2. Verwendung gemäß Anspruch 1, dadurch **gekennzeichnet**, daß die Salze aus Hydrochloriden oder Hydrobromiden ausgewählt sind.

3. Verwendung von N-Ethyl-5,6-dihydroxyindolin und N-Butyl-5,6-dihydroxyindolin zur Färbung keratinischer Fasern.

4. Färbezusammensetzung zur Verwendung zur Färbung keratinischer Fasern, insbesondere menschlicher keratinischer Fasern, dadurch **gekennzeichnet**, daß sie in einem zur Färbung geeigneten Milieu mindestens ein in jedem der Ansprüche 1 bis 3 definiertes 5,6-Dihydroxyindolin enthält.

5. Zusammensetzung gemäß Anspruch 4, dadurch **gekennzeichnet**, daß das 5,6-Dihydroxyindolin in der Zusammensetzung in Mengenverhältnissen von 0,01 bis 8 Gew.%, vorzugsweise von 0,03 bis 5 Gew.%, bezogen auf das Gesamtgewicht der Zusammensetzung, vorhanden ist.

6. Zusammensetzung gemäß Anspruch 4 oder 5, dadurch **gekennzeichnet**, daß das zur Färbung geeignete Milieu ein wässriges Milieu aus Wasser oder einer Mischung aus Wasser/Lösungsmittel(n) ist.

7. Zusammensetzung gemäß Anspruch 6, dadurch **gekennzeichnet**, daß die Lösungsmittel aus Ethylalkohol, Propyl- oder Isopropylalkohol, t-Butylalkohol, Ethylenglycol, Monomethyl-, Monoethyl- und Monobutylethern von Ethylenglycol, Monoethyletherethylenglycolacetat, Propylenglycol, Monomethylethern von Propylenglycol oder Dipropylenglycol, Methyllactat ausgewählt sind.

8. Färbezusammensetzung zur Färbung keratinischer Fasern und insbesondere menschlicher keratinischer Fasern, dadurch **gekennzeichnet**, daß sie, in einem zur Färbung geeigneten Milieu, das Wasser und mindestens ein Lösungsmittel umfaßt, ausgewählt aus Ethylalkohol, Propyl- oder Isopropylalkohol, t-Butylalkohol, Ethylenglycol, Monomethyl-, Monoethyl- und Monobutylethern von Ethylenglycol, Monoethyletherethylenglycolacetat, Propylenglycol, Monomethylethern von Propylenglycol und Dipropylenglycol und aus Methyllactat, mindestens eine Verbindung der Formel:

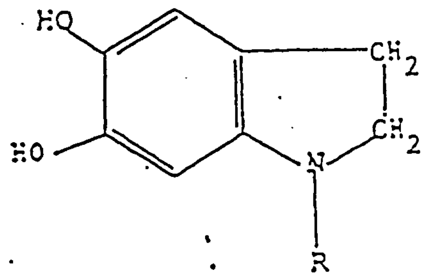

worin R einen $C_{1-4}$-Alkylrest bedeutet, sowie die Säureadditionssalze dieser Verbindungen enthält.

**9.** Zusammensetzung gemäß jedem der Ansprüche 4 bis 8,
dadurch **gekennzeichnet**, daß
die Zusammensetzung Fettamide, anionische, kationische, nicht-ionische, amphotere oberflächenaktive Mittel oder deren Mischungen, Verdickungsmittel, Parfüm-Produkte, Sequestriermittel, filmbildende Mittel, Behandlungsmittel, Dispergiermittel, Konditioniermittel, Konservierungsmittel, opak machende Mittel, Mittel zur Auflockerung keratinischer Fasern oder deren Mischungen enthält.

**10.** Zusammensetzung gemäß jedem der Ansprüche 4 bis 9,
dadurch **gekennzeichnet**, daß
der pH der Zusammensetzung 3 bis 12 beträgt.

**11.** Verfahren zur Färbung keratinischer Fasern, insbesondere menschlicher keratinischer Fasern,
dadurch **gekennzeichnet**, daß
man auf die Fasern mindestens eine Zusammensetzung aufträgt, die in einem zur Färbung geeigneten Milieu mindestens ein 5,6-Dihydroxyindolin der Formel (I'):

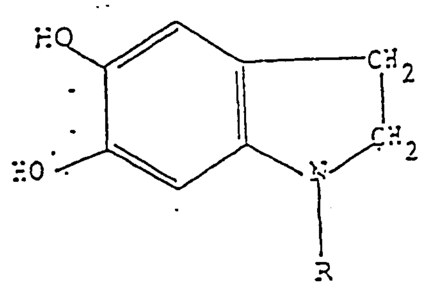

(I')

worin R Wasserstoff oder eine $C_{1-4}$-Alkylgruppe darstellt, sowie die Säureadditionssalze dieser Verbindungen enthält, und daß man diese Zusammensetzung in Kontakt mit den Fasern während einer Zeitdauer hält, die zur Entwicklung einer Färbung ausreicht, und zwar entweder an der Luft oder mittels eines chemischen oxidierenden Systems, das dargestellt ist durch:

(i) Jodidionen und Wasserstoffperoxid, wobei die Zusammensetzung (A) in diesem Fall, zusätzlich, entweder (a) Jodidionen oder (b) Wasserstoffperoxid enthält, und wobei vor oder nach der Aufbringung der Zusammensetzung (A) die Aufbringung einer Zusammensetzung (B) erfolgt, die in einem zur Färbung geeigneten Milieu entweder:

(a) Wasserstoffperoxid bei einem pH von 2 bis 12, wenn die Zusammensetzung (A) Jodidionen enthält, oder:

(b) Jodidionen bei einem pH von 3 bis 11 enthält, wenn die Zusammensetzung (A) Wasserstoffperoxid enthält;

(ii) Nitrite, wobei nach der Aufbringung der Zusammensetzung (A) die Aufbringung einer wässrigen Zusammensetzung (B) erfolgt, die einen sauren pH aufweist, wobei die Zusammensetzung (A) oder die Zusammensetzung (B) mindestens ein Nitrit enthalten;

(iii) Oxidationsmittel aus Wasserstoffperoxid, Periodsäure und ihren wasserlöslichen Salzen, Natriumhyypochlorit, Chloramin T, Chloramin B, Kaliumferricyanid, Silberoxid, Fenton-Reagens, Blei(IV)oxid, Cäsiumsulfat, Ammoniumpersulfat, wobei diese Oxidationsmittel in der Zusammensetzung (A) vorliegen, oder gleichzeitig oder anschließend mittels einer Zusammensetzung (B) aufgebracht werden, welche jene in einem zur Färbung geeigenten Milieu enthält;

(iv) Metallanionen aus Permanganaten oder Bichromaten, wobei diese Oxidationsmittel mittels einer wässrigen Zusammensetzung (B) bei einem pH von 2 bis 10 vor der Aufbringung der Zusammensetzung (A) aufgebracht werden;

(v) Metallsalze der Gruppen 3 bis 8 des Periodensystems, wobei die Metallsalze in einer getrennten Stufe mittels einer Zusammensetzung (B) aufgebracht werden, die diese Salze in einem zur Färbung geeigneten Milieu enthält;

(vi) seltene Erdmetallsalze, wobei die Salze der seltenen Erden mittels einer Zusammensetzung (B) aufgebracht werden, die jene in einem zur Färbung geeigneten Milieu enthält, wobei die Zusammensetzung (B) vor oder nach der Aufbringung der Zusammensetzung (A) aufgebracht wird;

(vii) ein Chinonderivat aus o- oder p-Benzochinonen, o- oder p-Benzochinonmonoiminen oder -diiminen, 1,2- oder 1,4-Naphthochinonen, o- oder p-Benzochinonsulfonimiden, α- ω-Alkylenbis-1,4-benzochinonen oder 1,2- oder 1,4-Naphthochinonmonoiminen oder -diiminen, wobei das 5,6-Dihydroxyindolin der Formel (I') und die Chinonderivate so ausgewählt sind, daß die Redox-Potentialdifferenz ΔE zwischen dem Redox-Potential $E_i$ des 5,6-Dihydroxyindolins der Formel (I'), bestimmt bei pH 7 in einem Phosphat-Milieu durch Voltametrie an einer Elektrode aus glasartigem Kohlenstoff, und dem Redox-Potential $E_q$ des Chinonderivats, bestimmt bei pH 7 in einem Phosphat-Milieu durch Polarographie an einer Hg-Elektrode gegen eine gesättigte Kalomel-Elektrode, beträgt:

$$\Delta E = E_i - E_q \leq 320 \text{ Millivolt},$$

wobei die Aufbringung der Zusammensetzung (B) vor oder nach der Aufbringung der Zusammensetzung (A) erfolgt, mit der Maßgabe, daß, wenn die Verbindung der Formel (I') 5,6-Dihydroxyindolin oder N-Methyl-5,6-dihydroxyindolin oder eines seiner Säureadditionssalze ist, das oxidierende System verschieden von Luft, Wasserstoffperoxid, Perjodsäure und ihren wasserlöslichen Salzen, von Ammonium- oder Kaliumpersulfat ist.

**12.** Verfahren zur Färbung keratinischer Fasern, insbesondere menschlicher keratinischer Fasern, dadurch **gekennzeichnet**, daß man auf diese Fasern mindestens eine Zusammensetzung aufträgt, die in einem zur Färbung geeigneten Milieu, das Wasser und mindestens ein Lösungsmittel oder alleiniglich ein oder mehrere wasserfreie Lösungsmittel umfaßt, wobei diese Lösungsmittel aus Ethylalkohol, Propyl- oder Isopropylalkohol, t-Butylalkohol, Ethylenglycol, Monomethyl-, Monoethyl- und Monobutylethern von Ethylenglycol, Monoethyletherethylenglycolacetat, Propylenglycol, Monomethylethern von Propylenglycol und Dipropylenglycol und aus Methyllactat ausgewählt sind, mindestens ein 5,6-Dihydroxyindolin der Formel (I'):

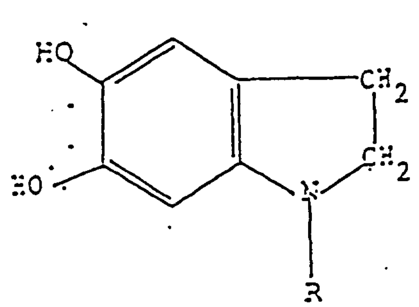

(I')

worin R Wasserstoff oder eine $C_{1-4}$-Alkylgruppe darstellt, sowie die Säureadditionssalze dieser Verbindungen enthält, und daß man diese Zusammensetzung in Kontakt mit den Fasern während einer Zeitdauer hält, die zur Entwicklung einer Färbung ausreicht, und zwar entweder an der Luft oder mittels eines chemischen oxidierenden Systems, das dargestellt ist durch:

(i) Jodidionen und Wasserstoffperoxid, wobei die Zusammensetzung (A) in diesem Fall, zusätzlich, entweder (a) Jodidionen oder (b) Wasserstoffperoxid enthält, und wobei vor oder nach der Aufbringung der Zusammensetzung (A) die Aufbringung einer Zusammensetzung (B) erfolgt, die in einem zur Färbung geeigneten Milieu entweder:

(a) Wasserstoffperoxid bei einem pH von 2 bis 12, wenn die Zusammensetzung (A) Jodidionen enthält, oder:

(b) Jodidionen bei einem pH von 3 bis 11 enthält, wenn die Zusammensetzung (A) Wasserstoffperoxid enthält;

(ii) Nitrite, wobei nach der Aufbringung der Zusammensetzung (A) die Aufbringung einer wässrigen Zusammensetzung (B) erfolgt, die einen sauren pH aufweist, wobei die Zusammensetzung (A) oder die Zusammensetzung (B) mindestens ein Nitrit enthalten;

(iii) Oxidationsmittel aus Wasserstoffperoxid, Perjodsäure und ihren wasserlöslichen Salzen, Natriumhyypochlorit, Chloramin T, Chloramin B, Kaliumferricyanid, Silberoxid, Fenton-Reagens, Blei(IV)oxid, Cäsiumsulfat, Ammoniumpersulfat, wobei diese Oxidationsmittel in der Zusammensetzung (A) vorliegen, oder gleichzeitig oder anschließend mittels einer Zusammensetzung (B) aufgebracht werden, welche jene in einem zur Färbung geeigenten Milieu enthält;

(iv) Metallanione aus Permanganaten oder Bichromaten, wobei diese Oxidationsmittel mittels einer wässrigen Zusammensetzung (B) bei einem pH von 2 bis 10 vor der Aufbringung der Zusammensetzung (A) aufgebracht werden;

(v) Metallsalze der Gruppen 3 bis 8 des Periodensystems, wobei die Metallsalze in einer getrennten Stufe mittels einer Zusammensetzung (B) aufgebracht werden, die diese Salze in einem zur Färbung geeigneten Milieu enthält;

(vi) seltene Erdmetallsalze, wobei die Salze der seltenen Erden mittels einer Zusammensetzung (B) aufgebracht werden, die jene in einem zur Färbung geeigneten Milieu enthält, wobei die Zusammensetzung (B) vor oder nach der Aufbringung der Zusammensetzung (A) aufgebracht wird;

(vii) ein Chinonderivat aus o- oder p-Benzochinonen, o- oder p-Benzochinonmonoiminen oder -diiminen, 1-2, oder 1,4-Naphthochinonen, o- oder p-Benzochinonsulfonimiden, $\alpha$- $\omega$-Alkylenbis-1,4-benzochinonen oder 1,2- oder 1,4-Naphthochinonmonoiminen oder -diiminen, wobei das 5,6-Dihydroxyindolin der Formel (I') und die Chinonderivate so ausgewählt sind, daß die Redox-Potentialdifferenz $\Delta E$ zwischen dem Redox-Potential $E_i$ des 5,6-Dihydroxyindolins der Formel (I'), bestimmt bei pH 7 in einem Phosphat-Milieu durch Voltametrie an einer Elektrode aus glasartigem Kohlenstoff, und dem Redox-Potential $E_q$ des Chinonderivats, bestimmt bei pH 7 in einem Phosphat-Milieu durch Polarographie an einer Hg-Elektrode gegen eine gesättigte Kalomel-Elektrode, beträgt:

$$\Delta E = E_i - E_q \leq 320 \text{ Millivolt,}$$

wobei die Zusammensetzung (B) vor oder nach der Zusammensetzung (A) aufgebracht wird.

**13.** Verfahren gemäß Anspruch 11 oder 12,
dadurch **gekennzeichnet**, daß
man die Färbung sich entwickeln läßt durch Kontakt an der Luft, ohne ein äußeres oxidierendes Mittel zuzufügen.

**14.** Verfahren gemäß Anspruch 11 oder 12,
dadurch **gekennzeichnet**, daß
man auf die keratinischen Materien eine Zusammensetzung (A) aufträgt, die in einem zur Färbung geeigneten

Milieu mindestens ein 5,6-Dihydroxyindolin der Formel (I') zusammen mit Jodidionen enthält, wobei die Aufbringung der Zusammensetzung (A) vor oder nach der Aufbringung einer Zusammensetzung (B) erfolgt, die in einem zur Färbung geeigneten Milieu Wasserstoffperoxid enthält.

**15.** Verfahren gemäß Anspruch 11 oder 12,
dadurch **gekennzeichnet**, daß
man auf die keratinischen Fasern mindestens eine Zusammensetzung (A) aufträgt, die in einem zur Färbung geeigneten Milieu mindestens ein 5,6-Dihydroxyindolin der Formel (I') zusammen mit Wasserstoffperoxid enthält und einen pH von 2 bis 7 aufweist, wobei die Aufbringung der Zusammensetzung (A) vor oder nach der Aufbingung einer Zusammensetzung (B) erfolgt, die in einem zur Färbung geeigneten Milieu Jodidionen enthält.

**16.** Verfahren gemäß Anspruch 14 oder 15,
dadurch **gekennzeichnet**, daß
die Jodidionen in der Zusammensetzung (A) oder (B) in Mengenverhältnissen von 0,007 bis 4 Gew.%, ausgedrückt in $J^-$-Ionen, bezogen auf Gesamtgewicht der Zusammensetzung (A) oder (B), vorliegen.

**17.** Verfahren gemäß Anspruch 11 oder 12,
dadurch **gekennzeichnet**, daß
man auf die keratinischen Materien eine Zusammensetzung (A) aufträgt, die das 5,6-Dihydroxyindolin (I') enthält, und daß man dann eine saure wässrige Zusammensetzung (B) aufbringt, wobei die Zusammensetzung (A) oder die Zusammensetzung (B) mindestens ein Nitrit enthalten, ausgewählt aus Alkali-, Erdalkalimetall-oder Ammoniumnitriten oder aus Nitriten eines jeden weiteren kosmetisch verträglichen Kations, aus einem organischen Nitritderivat und aus Vektoren von Nitrit, die ein Nitrit des oben definierten Typs erzeugen.

**18.** Verfahren gemäß Anspruch 17,
dadurch **gekennzeichnet**, daß
die Nitrite in Mengenverhältnissen von 0,02 bis 1 Mol/l vorhanden sind.

**19.** Verfahren gemäß Anspruch 11 oder 12,
dadurch **gekennzeichnet**, daß
man auf die keratinischen Fasern eine Zusammensetzung (B) aufbringt, die in einem zur Färbung geeigneten Milieu bei einem pH von 2 bis 10 ein Metallanion mit einer guten Affinität für Keratin enthält, ausgewählt aus Permanganaten oder Bichromaten, und daß man zu einem zweiten Zeitpunkt eine Zusammensetzung (A) aufträgt, die in einem zur Färbung geeigneten Milieu bei einem pH von 4 bis 10 ein 5,6-Dihydroxyindolin der Formel (I') enthält.

**20.** Verfahren gemäß Anspruch 19,
dadurch **gekennzeichnet**, daß
die Permanganate oder Bichromate in Molalitäten an Anionen oberhalb $10^{-3}$ Mol/1000 g bis vorzugsweise 1 Mol/1000 g verwendet werden, und daß die Zusammensetzungen kein organisches Agens enthalten, das eine reduzierende Wirkung auf die Anionen aufweist.

**21.** Vefahren gemäß Anspruch 11 oder 12,
dadurch **gekennzeichnet**, daß
man auf die keratinischen Fasern eine Zusammensetzung (A) aufträgt, die in einem zur Färbung geeigneten Milieu ein 5,6-Dihydroxyindolin der Formel (I') enthält, und daß man vor oder nach der Zusammensetzung (A) eine Zusammensetzung (B) aufbringt, die in einem zur Färbung geeigneten Milieu ein Metallsalz enthält, ausgewählt aus Salzen von Mangan, Kobalt, Eisen, Kupfer und Silber.

**22.** Verfahren gemäß Anspruch 21,
dadurch **gekennzeichnet**, daß
die Metallsalze in Mengenverhältnissen von 0,01 bis 2 Gew.%, ausgedrückt in Metallionen, bezogen auf Gesamtgewicht der Zusammensetzung, verwendet werden.

**23.** Verfahren gemäß Anspruch 11 oder 12,
dadurch **gekennzeichnet**, daß
man eine Zusammensetzung (A) aufträgt, die in einem zur Färbung geeigneten Milieu mindestens ein 5,6-Dihydroxyindolin der Formel (I') enthält, und daß man vor oder nach dieser Zusammensetzung eine Zusammensetzung (B) aufbringt, die in einem zur Färbung geeigneten Milieu ein Salz der seltenen Erden enthält, ausgewählt aus Salzen

von Cer, Lanthan, Europium, Gadolinium, Ytterbium, Dysprosium.

**24.** Verfahren gemäß Anspruch 23,
dadurch **gekennzeichnet**, daß
die Salze der seltenen Erden in Mengenverhältnissen von 0,1 bis 8 Gew.%, bezogen auf Gesamtgewicht der Zusammensetzung, vorhanden sind.

**25.** Verfahren gemäß Anspruch 11 oder 12,
dadurch **gekennzeichnet**, daß,
wenn man als oxidierendes Mittel eine Zusammensetzung auf Basis von Wasserstoffperoxid verwendet, der Gehalt an Wasserstoffperoxid in der Zusammensetzung 1 bis 40 Volumina, vorzugsweise 2 bis 10 Volumina, beträgt.

**26.** Mittel aus mehreren Bestandteilen zur Färbung keratinischer Fasern, insbesondere menschlicher keratinischer Fasern,
dadurch **gekennzeichnet**, daß
es einen ersten Bestandteil aus einer Zusammensetzung (A), enthaltend ein in Anspruch 11 definiertes 5,6-Dihydroxyindolin der Formel (I'), und einen zweiten Bestandteil aus einer der in jedem der Ansprüche 11 bis 25 definierten Zusammensetzungen (B) umfaßt, mit der Maßgabe, daß, wenn die Verbindung der Formel (I') 5,6-Dihydroxyindolin oder N-Methyl-5,6-dihydroxyindolin oder eines von deren Säureadditionssalzen ist, das oxidierende System verschieden von Wasser, Wasserstoffperoxid, Perjodsäure und ihren wasserlöslichen Salzen, von Ammonium- oder Kaliumpersulfat ist.

**27.** Mittel zur Färbung keratinischer Fasern, insbesondere menschlicher keratinischer Fasern, aus mehreren Teilen,
dadurch **gekennzeichnet**, daß
es einen ersten Bestandteil aus einer Zusammensetzung (A), die ein 5,6-Dihydroxyindolin der Formel (I'):

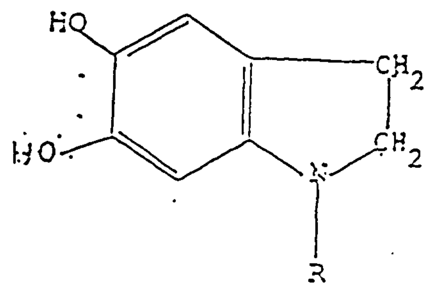

(I')

worin R Wasserstoff oder eine $C_{1-4}$-Alkylgruppe darstellt, sowie die Säureadditionssalze dieser Verbindungen in einem Milieu enthält, umfassend Wasser und mindestens ein Lösungsmittel oder alleiniglich ein oder mehrere wasserfreie Lösungsmittel, wobei diese Lösungsmittel aus Ethylalkohol, Propyl- oder Isopropylalkohol, t-Butylalkohol, Ethylenglycol, Monomethyl-, Monoethyl- und Monobutylethern von Ethylenglycol, Monoethyletherethylenglycolacetat, Propylenglycol, Monomethylethern von Propylenglycol und Dipropylenglycol und aus Methyllactat ausgewählt sind, und einen zweiten Bestandteil aus einem oxidierenden chemischen System umfaßt, ausgewählt aus:

(i) einer Zusammensetzung (B), die in einem zur Färbung geeigneten Milieu entweder:

a) Wasserstoffperoxid bei einem pH von 2 bis 12, wenn die Zusammensetzung (A) Jodidionen enthält, oder

b) Jodidionen bei einem pH von 3 bis 11 enthält, wenn die Zusammensetzung (A) Wasserstoffperoxid enthält;

(ii) einer wässrigen Zusammensetzung (B), die einen sauren pH-Wert aufweist, wenn die Zusammensetzung (A) ein Nitrit enthält, oder einer wässrigen Zusammensetzung (B) bei einem sauren pH-Wert, die mindestens ein Nitrit enthält;

(iii) einer Zusammensetzung (B), enthaltend ein Oxidiermittel, ausgewählt aus Wasserstoffperoxid, Perjodsäure und ihren wasserlöslichen Salzen, Natriumhypochlorit, Chloramin T, Chloramin B, Kaliumferricyanid, Silberoxid, Fenton-Reagens, Blei(IV)oxid, Cäsiumsulfat und aus Ammoniumpersulfat, in einem zur Färbung geeigneten Milieu;

(iv) einer wässrigen Zusammensetzung (B), enthaltend Metallanionen, ausgewählt aus Permanganat oder Bichromat, bei einem pH von 2 bis 10;

(v) einer Zusammensetzung (B), enthaltend Metallsalze der Gruppe III bis VIII des Periodensystems in einem zur Färbung geeigneten Milieu;

(vi) einer Zusammensetzung (B), enthaltend Salze seltener Erden in einem zur Färbung geeigneten Milieu;

(vii) einer Zusammensetzung (B), enthaltend ein Chinonderivat, ausgewählt aus o- oder p-Benzochinonen, o- oder p-Benzochinonmonoiminen oder -diiminen, 1,2- oder 1,4-Naphthochinonen, o- oder p-Benzochinonsulfonimiden, $\alpha,\omega$-Alkylenbis-1,4-benzochinonen oder 1,2- oder 1,4-Naphthochinonmonoiminen, wobei das 5,6-Dihydroxyindolin der Formel (I') der Zusammensetzung (A) und die Chinonderivate der Zusammensetzung (B) so ausgewählt sind, daß die Differenz des Redox-Potentials $\Delta E$ zwischen dem Redox-Potential $E_i$ des 5,6-Dihydroxyindolins der Formel (I'), bestimmt bei pH 7 in einem Phosphat-Milieu durch Voltametrie an einer Elektrode aus glasartigem Kohlenstoff, und dem Redox-Potential $E_q$ des Chinonderivats, bestimmt bei pH 7 in einem Phosphat-Milieu durch Polarographie an einer Hg-Elektrode gegen eine gesättigte Kalomel-Elektrode, beträgt:

$$\Delta E = E_i - E_q \leq 320 \text{ Millivolt.}$$

**28.** Mittel gemäß Anspruch 27,
dadurch **gekennzeichnet**, daß
die Zusammensetzung (A) in einem zur Färbung geeigneten Milieu mindestens ein 5,6-Dihydroxyindolin der Formel (I') zusammen mit Jodidionen und die Zusammensetzung (B) in einem zur Färbung geeigneten Mileiu Wasserstoffperoxid enthalten.

**29.** Mittel gemäß Anspruch 27,
dadurch **gekennzeichnet**, daß
die Zusammensetzung (A) in einem zur Färbung geeigneten Milieu mindestens ein 5,6-Dihydroxyindolin der Formel (I') zusammen mit Wasserstoffperoxid bei einem pH von 2 bis 7 und die Zusammensetzung (B) in einem zur Färbung geeigneten Milieu Jodidionen enthalten.

**30.** Mittel gemäß Anspruch 28 oder 29,
dadurch **gekennzeichnet**, daß
die Jodidionen in der Zusammensetzung (A) oder (B) in Mengenverhältnissen von 0,007 bis 4 Gew.%, ausgedrückt in Jodidionen, bezogen auf das Gesamtgewicht der Zusammensetzung (A) oder (B), vorhanden sind.

**31.** Mittel gemäß Anspruch 27,
dadurch **gekennzeichnet**, daß
die Zusammensetzung (A) das 5,6-Dihydroxyindolin der Formel (I') enthält und die Zusammensetzung (B) wässrig sauer ist, wobei die Zusammensetzung (A) oder die Zusammensetzung (B) mindestens ein Nitrit enthalten, ausgewählt aus Alkalimetall-, Erdalkali- oder Ammoniumnitriten oder aus jedem weiteren Nitrit eines kosmetisch geeigneten Kations, aus einem organischen Derivat von Nitrit und aus Vektoren von Nitrit, die ein Nitrit des oben definierten Typs erzeugen.

**32.** Mittel gemäß Anspruch 31,
dadurch **gekennzeichnet**, daß
die Nitrite in Mengenverhältnissen von 0,02 bis 1 Mol/l vorhanden sind.

**33.** Mittel gemäß Anspruch 27,
dadurch **gekennzeichnet**, daß
die Zusammensetzung (B) in einem zur Färbung geeigneten Milieu bei einem pH von 2 bis 10 einen Anion eines Metalls, das eine gute Affinität für Keratin aufweist, enthält, wobei das Anion aus Permanganat oder Bichromaten ausgewählt ist.

**34.** Mittel gemäß Anspruch 33,
dadurch **gekennzeichnet**, daß
die Permanganate oder Bichromate in Molalitäten an Anionen oberhalb $10^{-3}$ Mol/1000 g bis vorzugsweise 1 Mol/1000 g verwendet werden und die Zusammensetzungen kein organisches Mittel enthalten, das gegenüber den Anionen eine Reduzierwirkung aufweist.

**35.** Mittel gemäß Anspruch 27,
dadurch **gekennzeichnet**, daß
die Zusammensetzung (B) in einem zur Färbung geeigneten Milieu ein Metallsalz enthält, ausgewählt aus Salzen von Mangan, Kobalt, Eisen, Kupfer und Silber.

**36.** Mittel gemäß Anspruch 35,
dadurch **gekennzeichnet**, daß
die Metallsalze in Mengenverhältnissen von 0,01 bis 2 Gew.%, ausgedrückt in Metallionen, bezogen auf Gesamtgewicht der Zusammensetzung, verwendet werden.

**37.** Mittel gemäß Anspruch 27,
dadurch **gekennzeichnet**, daß
die Zusammensetzung (B) in einem zur Färbung geeigneten Milieu ein Salz von seltenen Erden enthält, ausgewählt aus den Salzen von Cer, Lanthan, Europium, Gadolinium, Ytterbium und Dysprosium.

**38.** Mittel gemäß Anspruch 37,
dadurch **gekennzeichnet**, daß
die Salze der seltenen Erden in Mengenverhältnissen von 0,1 bis 8 Gew.%, bezogen auf Gesamtgewicht der Zusammensetzung, vorhanden sind.

**39.** Mittel gemäß Anspruch 27,
dadurch **gekennzeichnet**, daß
die Zusammensetzung (B) auf Wasserstoffperoxid beruht, wobei der Gehalt an Wasserstoffperoxid in der Zusammensetzung 1 bis 40 und vorzugsweise 2 bis 10 Volumina beträgt.

**40.** Vorrichtung aus mehreren Teilen oder "Kit zur Färbung",
dadurch **gekennzeichnet**, daß
die verschiedenen Teile die verschiedenen Bestandteile des in jedem der Ansprüche 26 bis 39 definierten Färbemittels enthalten.